# EUROPÄISCHE PATENTSCHRIFT

(11) **EP 0 785 926 B1**
(45) Veröffentlichungstag und Bekanntmachung des Hinweises auf die Patenterteilung: **22.08.2001**
(21) Anmeldenummer: 95935916.7
(22) Anmeldetag: 07.10.1995
(51) Int. Cl.: C07D 239/60, C07D 403/12, C07D 251/30, C07D 239/96, C07D 491/04, A61K 31/505

(54) **CARBONSÄUREDERIVATE, IHRE HERSTELLUNG UND VERWENDUNG ALS ARZNEIMITTEL**
CARBOXYLIC ACID DERIVATIVES, THEIR PREPARATION AND THEIR USE AS MEDICAMENTS
DERIVES D'ACIDE CARBOXYLIQUE, LEUR PREPARATION ET LEUR UTILISATION COMME MEDICAMENTS

(30) Priorität: 14.10.1994 DE 4436851; 07.09.1995 DE 19533023
(43) Veröffentlichungstag der Anmeldung: 30.07.1997
(60) Teilanmeldung: 01103889.0
(73) Patentinhaber: BASF AKTIENGESELLSCHAFT, 67056 Ludwigshafen (DE)
(72) Erfinder: RIECHERS, Hartmut, D-67435 Neustadt (DE); KLINGE, Dagmar, D-69120 Heidelberg (DE); AMBERG, Wilhelm, D-61381 Friedrichsdorf (DE); KLING, Andreas, D-68239 Mannheim (DE); MÜLLER, Stefan, D-67346 Speyer (DE); BAUMANN, Ernst, D-67373 Dudenhofen (DE); RHEINHEIMER, Joachim, D-67063 Ludwigshafen (DE); VOGELBACHER, Uwe, Josef, D-67071 Ludwigshafen (DE); WERNET, Wolfgang, D-67454 Hassloch (DE); UNGER, Liliane, D-67065 Ludwigshafen (DE); RASCHACK, Manfred, D-67256 Weisenheim (DE)
(86) Internationale Anmeldenummer: EP9503963
(87) Internationale Veröffentlichungsnummer: WO9611914

(56) Entgegenhaltungen:
- EP-A- 0 347 811
- EP-A- 0 481 512
- EP-A- 0 517 215
- DE-A- 4 313 412
- DE-A- 4 313 413
- DE-A- 4 335 950

## Beschreibung

Die vorliegende Erfindung betrifft neue Carbonsäuredrivate, deren Herstellung und Verwendung.

DE 43 13 413 und DE 43 13 412 betreffen 3-(Het)aryl-Carbonsäurederivate und ihre Verwendung als Herbizide. WO 95/26716 betrifft die Verwendung der in DE 43 13 412 genannten Verbindungen als Arzneimittel, insbesondere als Hemmstoffe für Endothelinrezeptoren.

Endothelin ist ein aus 21 Aminosäuren aufgebautes Peptid, das von vaskulärem Endothel synthetisiert und freigesetzt wird. Endothelin existiert in drei Isoformen, ET-1, ET-2 und ET-3.
Im Folgenden bezeichnet "Endothelin" oder "ET" eine oder alle Isoformen von Endothelin. Endothelin ist ein potenter Vasokonstriktor und hat einen starken Effekt auf den Gefäßtonus. Es ist bekannt, daß diese Vasokonstriktion von der Bindung von Endothelin an seinen Rezeptor verursacht wird (Nature, 332, 411-415, 1988; FEBS Letters, 231, 440-444, 1988 und Biochem. Biophys. Res. Commun., 154, 868-875, 1988).

Erhöhte oder abnormale Freisetzung von Endothelin verursacht eine anhaltende Gefäßkontraktion in peripheren, renalen und zerebralen Blutgefäßen, die zu Krankheiten führen kann. Wie in der Literatur berichtet, wurden erhöhte Plasmaspiegel von Endothelin gefunden bei Patienten mit Hypertonie, akutem Myokardinfarkt, pulmonärer Hypertonie, Raynaud-Syndrom, Atherosklerose und in den Atemwegen von Asthmatikern (Japan J. Hypertension, 12, 79 (1989), J. Vascular Med. Biology 2, 207 (1990), J. Am. Med. Association 264, 2868 (1990)).

Demnach sollten Substanzen, die spezifisch die Bindung von Endothelin an den Rezeptor inhibieren, auch die obengenannten verschiedenen physiologischen Effekte von Endothelin antagonisieren und daher wertvolle Pharmaka darstellen.

Es wurde nun gefunden, daß bestimmte Carbonsäurederivate gute Hemmstoffe für Endothelinrezeptoren sind.

Gegenstand der Erfindung sind Carbonsäurederivate der Formel I 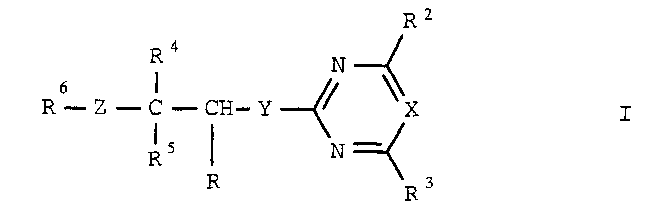 in der R Tetrazol, eine Gruppe COOH oder einen zu COOH hydrolysierbaren Rest bedeutet und die übrigen Substituenten folgende Bedeutung haben:
- R²: Wasserstoff, Hydroxy, NH₂, NH(C₁-C₄-Alkyl), N(C₁-C₄-Alkyl)₂, Halogen, C₁-C₄-Alkyl, C₁-C₄-Halogenalkyl, C₁-C₄-Alkoxy, C₁-C₄-Halogenalkoxy oder C₁-C₄-Alkylthio;
- X: Stickstoff oder CR¹⁴, worin R¹⁴ Wasserstoff oder C₁₋₅-Alkyl bedeutet oder CR¹⁴ zusammen mit CR³ einen 5- oder 6-gliedrigen Alkylen- oder Alkenylenring bildet, der durch eine oder zwei C₁₋₄-Alkylgruppen substituiert sein kann und worin jeweils eine Methylengruppe durch Sauerstoff, Schwefel, -NH oder -NC₁₋₄-Alkyl ersetzt sein kann;
- R³: Wasserstoff, Hydroxy, NH₂, NH(C₁-C₄-Alkyl), N(C₁-C₄-Alkyl)₂, Halogen, C₁-C₄-Alkyl, C₁-C₄-Halogenalkyl, C₁-C₄-Alkoxy, C₁-C₄-Halogenalkoxy, -NH-O-C₁₋₄-Alkyl, C₁-C₄-Alkylthio oder CR³ ist mit CR¹⁴ wie oben angegeben zu einem 5- oder 6-gliedrigen Ring verknüpft;
- R⁴: und R⁵ Phenyl oder Naphthyl, die durch einen oder mehrere der folgenden Reste substituiert sein können: Halogen, Nitro, Cyano, Hydroxy, C₁-C₄-Alkyl, C₁-C₄-Halogenalkyl, C₁-C₄-Alkoxy, C₁-C₄-Halogenalkoxy, Phenoxy, C₁-C₄-Alkylthio, Amino, C₁-C₄-Alkylamino oder C₁-C₄-Dialkylamino; oder
Phenyl oder Naphthyl, die orthoständig über eine direkte Bindung, eine Methylen-, Ethylen- oder Ethenylengruppe, ein Sauerstoff- oder Schwefelatom oder eine SO₂-, NH- oder N-Alkyl-Gruppe miteinander verbunden sind oder C₃-C₇-Cycloalkyl;
wobei R⁴ die gleiche Bedeutung wie R⁵ hat;
- R⁶: Wasserstoff, C₁-C₈-Alkyl, C₃-C₆-Alkenyl, C₃-C₆-Alkinyl oder C₃-C₈-Cycloalkyl, wobei diese Reste jeweils ein- oder mehrfach substituiert sein können durch: Halogen, Nitro, Cyano, C₁-C₄-Alkoxy, C₃-C₆-Alkenyloxy, C₃-C₆-Alkinyloxy, C₁-C₄-Alkylthio, C₁-C₄-Halogenalkoxy, C₁-C₄-Alkylcarbonyl, C₁-C₄-Alkoxycarbonyl, C₃₋₈-Alkylcarbonylalkyl, C₁-C₄-Alkylamino, Di-C₁-C₄-alkylamino, Phenyl oder ein- oder mehrfach, durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio substituiertes Phenyl oder Phenoxy;
Phenyl oder Naphthyl, die jeweils durch einen oder mehrere der folgenden Reste substituiert sein können: Halogen, Nitro, Cyano, Hydroxy, Amino, C₁-C₄-Alkyl, C₁-C₄-Halogenalkyl, C₁-C₄-Alkoxy, C₁-C₄-Halogenalkoxy, Phenoxy, C₁-C₄-Alkylthio, C₁-C₄-Alkylamino, C₁-C₄-Dialkylamino, Dioxomethylen oder Dioxoethylen;
ein fünf- oder sechsgliedriger Heteroaromat, enthaltend ein bis drei Stickstoffatome und/oder ein Schwefel- oder Sauerstoffatom, welcher ein bis vier Halogenatome und/oder einen bis zwei der folgenden Reste tragen kann: C₁-C₄-Alkyl, C₁-C₄-Halogenalkyl, C₁-C₄-Alkoxy, C₁-C₄-Halogenalkoxy, C₁-C₄-Alkylthio, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
mit der Maßgabe, daß R⁶ nur dann Wasserstoff bedeuten kann, wenn Z keine Einfachbindung darstellt;
- Y: Sauerstoff;
- z: Schwefel, Sauerstoff, -SO- oder -SO₂.

Die Verbindungen und auch die Zwischenprodukte zu ihrer Herstellung, wie z.B. IV und VI, können ein oder mehrere asymmetrische substituierte Kohlenstoffatome besitzen. Solche Verbindungen können als reine Enantiomere bzw. reine Diastereomere oder als deren Mischung vorliegen. Bevorzugt ist die Verwendung einer enantiomerenreinen Verbindung als Wirkstoff.

Gegenstand der Erfindung ist weiter die Verwendung der oben genannten Carbonsäurederivate zur Herstellung von Arzneimitteln, insbesondere zur Herstellung von Hemmstoffen für Endothelinrezeptoren.

Ein weiterer Gegenstand der Erfindung ist die Herstellung von Verbindungen der Formel IV in enantiomerenreiner Form. Die enantioselektive Epoxidierung eines zweifach Phenyl-substituierten Olefins ist bekannt (J. Org. Chem. 1994, 59, 4378-4380).

Überraschenderweise wurde nun gefunden, daß auch Estergruppen in diesen Systemen eine Epoxidierung in hoher optischer Reinheit erlauben.

Die Herstellung der erfindungsgemäßen Verbindungen, in denen Z Schwefel oder Sauerstoff ist, geht aus von den Epoxiden IV, die man in allgemein bekannter Weise, z.B. wie in J. March, Advanced Organic Chemistry, 2nd ed., 1983, S. 862 und S. 750 beschrieben, aus den Ketonen II oder den Olefinen III erhält:

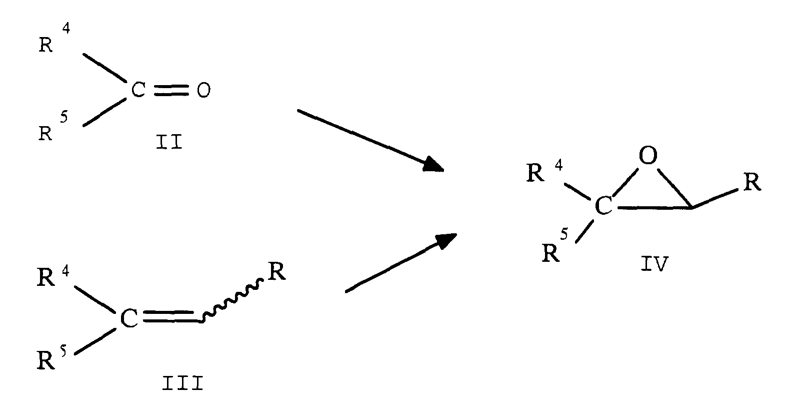

Carbonsäurederivate der allgemeinen Formel VI können hergestellt werden, indem man die Epoxide der allgemeinen Formel IV (z.B. mit R = ROOR¹⁰) mit Alkoholen oder Thiolen der allgemeinen Formel V, in der R⁶ und Z die in Anspruch 1 genannte Bedeutung haben, zur Reaktion bringt.

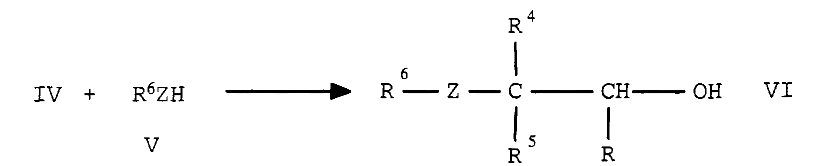

Dazu werden Verbindungen der allgemeinen Formel IV mit Verbindungen der Formel V, im Molverhältnis von etwa 1:1 bis 1:7, bevorzugt 1 bis 3 Moläquivalenten, auf eine Temperatur von 50 bis 200°C, bevorzugt 80 bis 150°C, erhitzt.

Die Reaktion kann auch in Gegenwart eines Verdünnungsmittels erfolgen. Zu diesem Zweck können sämtliche gegenüber den verwendeten Reagenzien inerte Lösungsmittel verwendet werden.

Beispiele für solche Lösungsmittel beziehungsweise Verdünnungsmittel sind Wasser, aliphatische, alicyclische und aromatische Kohlenwasserstoffe, die jeweils gegebenenfalls chloriert sein können, wie zum Beispiel Hexan, Cyclohexan, Petrolether, Ligroin, Benzol, Toluol, Xylol, Methylenchlorid, Chloroform, Kohlenstofftetrachlorid, Ethylchlorid und Trichlorethylen, Ether, wie zum Beispiel Diisopropylether, Dibutylether, Methyl-tert.-Butylether-Propylenoxid, Dioxan und Tetrahydrofuran, Ketone, wie zum Beispiel Aceton, Methylethylketon, Methylisopropylketon und Methylisobutylketon, Nitrile, wie zum Beispiel Acetonitril und Propionitril, Alkohole, wie zum Beispiel Methanol, Ethanol, Isopropanol, Butanol und Ethylenglycol, Ester, wie zum Beispiel Ethylacetat und Amylacetat, Säureamide, wie zum Beispiel Dimethylformamid, Dimethylacetamid und N-Methylpyrrolidon, Sulfoxide und Sulfone, wie zum Beispiel Dimethylsulfoxid und Sulfolan, Basen, wie zum Beispiel Pyridin, cyclische Harnstoffe wie 1,3-Dimethylimidazolidin-2-on und 1,3-Dimethyl-3,4,5,6-tetrahydro-2(1H)-pyrimidinon.

Die Reaktion wird dabei bevorzugt in einem Temperaturbereich zwischen 0°C und dem Siedepunkt des Lösungsmittels bzw. Lösungsmittelgemisches durchgeführt.

Die Gegenwart eines Reaktionskatalysators kann von Vorteil sein. Als Katalysatoren kommen dabei starke organische und anorganische Säuren sowie Lewissäuren in Frage. Beispiele hierfür sind unter anderem Schwefelsäure, Salzsäure, Trifluoressigsäure, p-Toluolsulfonsäure, Bortrifluorid-Etherat und Titan(IV)-Alkoholate.

Verbindungen der Formel VI, in denen R⁴ und R⁵ Cycloalkyl bedeutet, können auch dadurch hergestellt werden, daß man Verbindungen der Formel VI, in denen R⁴ und R⁵ Phenyl, Naphthyl oder wie oben beschrieben substituiertes Phenyl oder Naphthyl bedeutet, einer Kernhydrierung unterwirft.

Verbindungen der Formel VI können in enantiomerenreiner Form erhalten werden, indem man von enantiomerenreiner Verbindungen der Formel IV ausgeht und sie in beschriebener Weise mit Verbindungen der Formel V umsetzt.

Weiterhin kann man enantiomerenreine Verbindungen der Formel VI erhalten, indem man mit racemischen bzw. diastereomeren Verbindungen der Formel VI eine klassische Racematspaltung mit geeigneten enantiomerenreinen Basen wie z.B. Brucin, Strychnin, Quinin, Quinidin, Chinchonidin, Chinchonin, Yohimbin, Morphin, Dehydroabietylamin, Ephedrin (-), (+), Deoxyephedrin (+), (-), threo-2-Amino-1-(p-nitrophenyl)-1,3-propandiol (+), (-), threo-2-(N,N-Dimethylamino)-l-(p-nitrophenyl)-1,3-propandiol (+), (-) threo-2-Amino-l-phenyl-1,3-propandiol (+), (-), α-Methylbenzylamin (+), (-), α-(1-Naphthyl)ethylamin (+), (-), α-(2-Naphthyl)ethylamin (+), (-), Aminomethylpinan, N,N-Dimethyl-1-phenylethylamin, N-Methyl-1-phenylethylamin, 4-Nitrophenylethylamin, Pseudoephedrin, Norephedrin, Norpseudoephedrin, Aminosäurederivate, Peptidderivate durchführt.

Die erfindungsgemäßen Verbindungen, in denen Y Sauerstoff bedeutet und die restlichen Substituenten die unter der allgemeinen Formel I angegebenen Bedeutung haben, können beispielsweise derart hergestellt werden, daß man die Carbonsäurederivate der allgemeinen Formel VI, in denen die Substituenten die angegebene Bedeutung haben, mit Verbindungen der allgemeinen Formel VII, 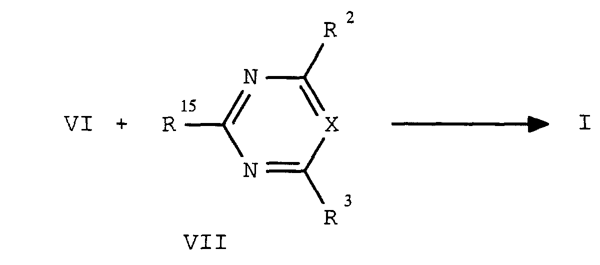 in der R¹⁵ Halogen oder R¹⁶-SO₂- bedeutet, wobei R¹⁶ C₁-C₄-Alkyl, C₁-C₄-Halogenalkyl oder Phenyl sein kann, zur Reaktion bringt. Die Reaktion findet bevorzugt in einem der oben genannten inerten Verdünnungsmittel unter Zusatz einer geeigneten Base, d.h. einer Base, die eine Deprotonierung des Zwischenproduktes VI bewirkt, in einem Temperaturbereich von Raumtemperatur bis zum Siedepunkt des Lösungsmittels statt.

Verbindungen der Formel VII sind bekannt, teilweise käuflich oder können nach allgemein bekannter Weise hergestellt werden.

Als Base kann ein Alkali- oder Erdalkalimetallhydrid wie Natriumhydrid, Kaliumhydrid oder Calciumhydrid, ein Carbonat wie Alkalimetallcarbonat, z.B. Natrium- oder Kaliumcarbonat, ein Alkali- oder Erdalkalimetallhydroxid wie Natrium- oder Kaliumhydroxid, eine metallorganische Verbindung wie Butyllithium oder ein Alkaliamid wie Lithiumdiisopropylamid dienen.

Die erfindungsgemäßen Verbindungen, in denen Y Schwefel bedeutet und die restlichen Substituenten die unter der allgemeinen Formel I angegebene Bedeutung haben, können beispielsweise derart hergestellt werden, daß man Carbonsäurederivate der allgemeinen Formel VIII, die in bekannter Weise aus Verbindungen der allgemeinen Formel VI erhältlich sind und in denen die Substituenten die oben angegebene Bedeutung haben, mit Verbindungen der allgemeinen Formel IX, in der R², R³ und X die unter der allgemeinen Formel I angegebene Bedeutung haben, zur Reaktion bringt.

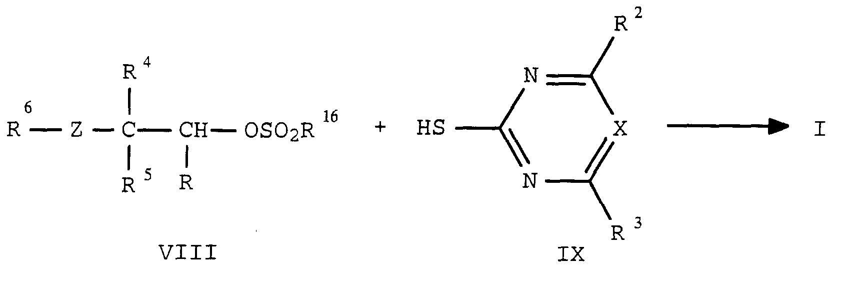

Die Reaktion findet bevorzugt in einem der oben genannten inerten Verdünnungsmittel unter Zusatz einer geeigneten Base, d.h. eine Base, die eine Deprotonierung des Zwischenproduktes IX bewirkt, in einem Temperaturbereich von Raumtemperatur bis zum Siedepunkt des Lösungsmittels statt.

Als Base können neben den oben genannten auch organische Basen wie Triethylamin, Pyridin, Imidazol oder Diazabicycloundecan dienen.

Verbindungen der Formel I können auch dadurch hergestellt werden, daß man von den entsprechenden Carbonsäuren, d. h. Verbindungen der Formel I, in denen R COOH bedeutet, ausgeht und diese zunächst auf übliche Weise in eine aktivierte Form wie ein Säurehalogenid, ein Anhydrid oder Imidazolid überführt und dieses dann mit einer entsprechenden Hydroxylverbindung HOR¹⁰ umsetzt. Diese Umsetzung läßt sich in den üblichen Lösungsmitteln durchführen und erfordert oft die Zugabe einer Base, wobei die oben genannten in Betracht kommen. Diese beiden Schritte lassen sich beispielsweise auch dadurch vereinfachen, daß man die Carbonsäure in Gegenwart eines wasserabspaltenden Mittels wie eines Carbodiimids auf die Hydroxylverbindung einwirken läßt.

Außerdem können Verbindungen der Formel I auch dadurch hergestellt werden, daß man von den Salzen der entsprechenden Carbonsäuren ausgeht, d. h. von Verbindungen der Formel I, in denen R für eine Gruppe COR¹ und R¹ für OM stehen, wobei M ein Alkalimetallkation oder das Äquivalent eines Erdalkalimetallkations sein kann. Diese Salze lassen sich mit vielen Verbindungen der Formel R¹-A zur Reaktion bringen, wobei A eine übliche nucleofuge Abgangsgruppe bedeutet, beispielsweise Halogen wie Chlor, Brom, Iod oder gegebenenfalls durch Halogen, Alkyl oder Halogenalkyl substituiertes Aryl- oder Alkylsulfonyl wie z.B. Toluolsulfonyl und Methylsulfonyl oder eine andere äquivalente Abgangsgruppe. Verbindungen der Formel R¹-A mit einem reaktionsfähigen Substituenten A sind bekannt oder mit dem allgemeinen Fachwissen leicht zu erhalten. Diese Umsetzung läßt sich in den üblichen Lösungsmitteln durchführen und wird vorteilhaft unter Zugabe einer Base, wobei die oben genannten in Betracht kommen, vorgenommen.

Der Rest R in Formel I ist breit variabel. Beispielsweise steht R für eine Gruppe 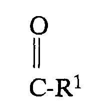 in der R¹ die folgende Bedeutung hat:
a) Wasserstoff;
b) eine Succinylimidoxygruppe;
c) ein über ein Stickstoffatom verknüpfter 5-gliedriger Heteroaromat wie Pyrrolyl, Pyrazolyl, Imidazolyl und Triazolyl, welcher ein bis zwei Halogenatome, insbesondere Fluor und Chlor und/oder ein bis zwei der folgenden Reste tragen kann:
   C₁-C₄-Alkyl wie Methyl, Ethyl, 1-Propyl, 2-Propyl, 2-Methyl-2-propyl, 2-Methyl-1-propyl, 1-Butyl, 2-Butyl;
   C₁-C₄-Halogenalkyl, insbesondere C₁-C₂-Halogenalkyl wie beispielsweise Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl;
   C₁-C₄-Halogenalkoxy, insbesondere C₁-C₂-Halogenalkoxy wie Difluormethoxy, Trifluormethoxy, Chlordifluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy;
   C₁-C₄-Alkoxy wie Methoxy, Ethoxy, Propoxy, 1-Methylethoxy, Butoxy, 1-Methylpropoxy, 2-Methylpropoxy, 1,1-Dimethylethoxy, insbesondere Methoxy, Ethoxy, 1-Methylethoxy;
   C₁-C₄-Alkylthio wie Methylthio, Ethylthio, Propylthio, 1-Methylethylthio, Butylthio, 1-Methylpropylthio, 2-Methylpropylthio, 1,1-Dimethylethylthio, insbesondere Methylthio und Ethylthio;
d) R¹ ferner ein Rest 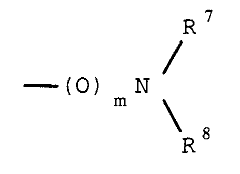 in dem m für 0 oder 1 steht und R⁷ und R⁸, die gleich oder unterschiedlich sein können, die folgende Bedeutung haben:
   Wasserstoff
   C₁-C₈-Alkyl, insbesondere C₁-C₄-Alkyl wie oben genannt;
   C₃-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-l-methyl-2-propenyl und 1-Ethyl-2-methyl-2-propenyl, insbesondere 2-Propenyl, 2-Butenyl, 3-Methyl-2-butenyl und 3-Methyl-2-pentenyl;
   C₃-C₆-Alkinyl wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-l-methyl-2-propinyl, vorzugsweise 2-Propinyl, 2-Butinyl, 1-Methyl-2-propinyl und 1-Methyl-2-butinyl, insbesondere 2-Propinyl
   C₃-C₈-Cycloalkyl, wie Cyclopropyl, Cyclobutyl, Cyclopentyl, Cyclohexyl und Cycloheptyl, Cyclooctyl, wobei diese Alkyl-, Cycloalkyl-, Alkenyl- und Alkinylgruppen jeweils ein bis fünf Halogenatome, insbesondere Fluor oder Chlor und/oder ein bis zwei der folgenden Gruppen tragen können:
   C₁-C₄-Alkyl, C₁-C₄-Alkoxy, C₁-C₄-Alkylthio, C₁-C₄-Halogenalkoxy wie vorstehend genannt, C₃-C₆-Alkenyloxy, C₃-C₆-Alkenylthio, C₃-C₆-Alkinyloxy, C₃-C₆-Alkinylthio, wobei die in diesen Resten vorliegenden Alkenyl- und Alkinylbestandteile vorzugsweise den oben genannten Bedeutungen entsprechen;
   C₁-C₄-Alkylcarbonyl wie insbesondere Methylcarbonyl, Ethylcarbonyl, Propylcarbonyl, 1-Methylethylcarbonyl, Butylcarbonyl, 1-Methylpropylcarbonyl, 2-Methylpropylcarbonyl, 1,1-Dimethylethylcarbonyl;
   C₁-C₄-Alkoxycarbonyl wie Methoxycarbonyl, Ethoxycarbonyl, Propyloxycarbonyl, 1-Methylethoxycarbonyl, Butyloxycarbonyl, 1-Methylpropyloxycarbonyl, 2-Methylpropyloxycarbonyl, 1,1-Dimethylethoxycarbonyl;
   C₃-C₆-Alkenylcarbonyl, C₃-C₆-Alkinylcarbonyl, C₃-C₆-Alkenyloxycarbonyl und C₃-C₆-Alkinyloxycarbonyl, wobei die Alkenyl- bzw. Alkinylreste vorzugsweise, wie voranstehend im einzelnen aufgeführt, definiert sind;
   Phenyl, gegebenenfalls ein- oder mehrfach, z.B. einbis dreifach substituiert durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio wie beispielsweise 2-Fluorphenyl, 3-Chlorphenyl, 4-Bromphenyl, 2-Methylphenyl, 3-Nitrophenyl, 4-Cyanophenyl, 2-Trifluormethylphenyl, 3-Methoxyphenyl, 4-Trifluorethoxyphenyl, 2-Methylthiophenyl, 2,4-Dichlorphenyl, 2-Methoxy-3-methylphenyl, 2,4-Dimethoxyphenyl, 2-Nitro-5-cyanophenyl, 2,6-Difluorphenyl;
   Di-C₁-C₄-Alkylamino wie insbesondere Dimethylamino, Dipropylamino, N-Propyl-N-methylamino, N-Propyl-N-ethylamino, Diisopropylamino, N-Isopropyl-N-methylamino, N-Isopropyl-N-ethylamino, N-Isopropyl-N-propylamino;
   R⁷ und R⁸ ferner Phenyl, das durch einen oder mehrere, z.B. ein bis drei der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio, wie insbesondere oben genannt;
   oder R⁷ und R⁸ bilden gemeinsam eine zu einem Ring geschlossene, optionell substituierte, z.B. durch C₁-C₄-Alkyl substituierte C₄-C₇-Alkylenkette, die ein Heteroatom, ausgewählt aus der Gruppe Sauerstoff, Schwefel oder Stickstoff, enthalten kann wie-(CH₂)₄-, -(CH₂)₅-, -(CH₂)₆-, -(CH₂)₇-, -(CH₂)₂-O-(CH₂)₂-, -CH₂-S-(CH₂)₃-, -(CH₂)₂-O-(CH₂)₃-, -NH-(CH₂)₃-, -CH₂-NH-(CH₂)₂-, -CH₂-CH=CH-CH₂-, -CH=CH-(CH₂)₃-;
e) R¹ ferner eine Gruppe 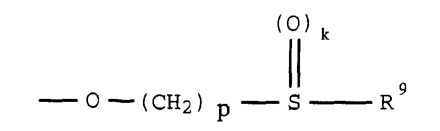 in der k die Werte 0, 1 und 2, p die Werte 1, 2, 3 und 4 annehmen und R⁹ für
   C₁-C₄-Alkyl, C₁-C₄-Halogenalkyl, C₃-C₆-Alkenyl, C₃-C₆-Alkinyl oder gegebenenfalls substituiertes Phenyl steht, wie insbesondere oben genannt.
f) R¹ ferner ein Rest OR¹⁰, worin R¹⁰ bedeutet:
   Wasserstoff, das Kation eines Alkalimetalls wie Lithium, Natrium, Kalium oder das Kation eines Erdalkalimetalls wie Calcium, Magnesium und Barium oder ein umweltverträgliches organisches Ammoniumion wie tertiäres C₁-C₄-Alkylammonium oder das Ammoniumion;
   C₃-C₈-Cycloalkyl wie vorstehend genannt, welches ein bis drei C₁-C₄-Alkylgruppen tragen kann;
   C₁-C₈-Alkyl wie insbesonder Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2 -Trimethylpropyl, 1,2,2-Trimethylpropyl, 1-Ethylbutyl, 2-Ethylbutyl, 1-Ethyl-2-methylpropyl, welches ein bis fünf Halogenatome, insbesondere Fluor und Chlor und/oder einen der folgenden Reste tragen kann:
   C₁-C₄-Alkoxy, C₁-C₄-Alkylthio, Cyano, C₁-C₄-Alkylcarbonyl, C₃-C₈-Cycloakyl, C₁-C₄-Alkoxycarbonyl, Phenyl, Phenoxy oder Phenylcarbonyl, wobei die aromatischen Reste ihrerseits jeweils ein bis fünf Halogenatome und/oder ein bis drei der folgenden Reste tragen können: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio, wie insbesondere oben genannt;
   eine C₁-C₈-Alkylgruppe wie vorstehend genannt, welch ein bis fünf Halogenatome, insbesonder Fluor und/oder Chlor tragen kann und einen der folgenden Reste trägt: ein 5-gliedriger Heteroaromat, enthaltend ein bis drei Stickstoffatome, oder ein 5-gliedriger Heteroaromat enthaltend ein Stickstoffatom und ein Sauerstoff- oder Schwefelatom, welcher ein bis vier Halogenatome und/oder ein bis zwei der folgenden Reste tragen kann:
   Nitro, Cyano, C₁-C₄-Alkyl, C₁-C₄-Halogenalkyl, C₁-C₄-Alkoxy, Phenyl, C₁-C₄-Halogenalkoxy und/oder C₁-C₄-Alkylthio. Insbesondere seien genannt: 1-Pyrazolyl, 3-Methyl-1-pyrazolyl, 4-Methyl-1-pyrazolyl, 3,5-Dimethyl-l-pyrazolyl, 3-Phenyl-1-pyrazolyl, 4-Phenyl-1-pyrazolyl, 4-Chlor-1-pyrazolyl, 4-Brom-1-pyrazolyl, 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl, 3-Isopropylisoxazol-5-yl, 3-Methylisoxazol-5-yl, Oxazol-2-yl, Thiazol-2-yl, Imidazol-2-yl, 3-Ethylisoxazol-5-yl, 3-Phenylisoxazol-5-yl, 3-tert.-Butylisoxazol-5-yl; eine C₂-C₆-Alkylgrupe, welche in der 2-Position einen der folgenden Reste trägt: C₁-C₄-Alkoxyimino, C₃-C₆-Alkinyloxyimino, C₃-C₆-Halogenalkenyloxyimino oder Benzyloxyimino;
   eine C₃-C₆-Alkenyl- oder eine C₃-C₆-Alkinylgruppe, wobei diese Gruppen ihrerseits ein bis fünf Halogenatöme tragen können;
   R¹⁰ ferner ein Phenylrest, welcher ein bis fünf Halogenatome und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio, wie insbesondere oben genannt;
   ein über ein Stickstoffatom verknüpfter 5-gliedriger Heteroaromat, enthaltend ein bis drei Stickstoffatome, welcher ein bis zwei Halogenatome und/oder ein bis zwei der folgenden Reste tragen kann: C₁-C₄-Alkyl, C₁-C₄-Halogenalkyl, C₁-C₄-Alkoxy, Phenyl, C₁-C₄-Halogenalkoxy und/oder C₁-C₄-Alkylthio. Insbesondere seien genannt: 1-Pyrazolyl, 3-Methyl-1-pyrazolyl, 4-Methyl-1-pyrazolyl, 3,5-Dimethyl-l-pyrazolyl, 3-Phenyl-1-pyrazolyl, 4-Phenyl-1-pyrazolyl, 4-Chlor-1-pyrazolyl, 4-Brom-1-pyrazolyl, 1-Imidazolyl, 1-Benzimidazolyl, 1,2,4-Triazol-1-yl, 3-Methyl-1,2,4-triazol-1-yl, 5-Methyl-1,2,4-triazol-1-yl, 1-Benztriazolyl, 3,4-Dichlorimidazol-1-yl;
   R¹⁰ ferner ein Gruppe 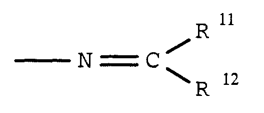 worin R¹¹ und R¹², die gleich oder verschieden sein können, bedeuten:
      C₁-C₈-Alkyl, C₃-C₆-Alkenyl, C₃-C₆-Alkinyl, C₃-C₈-Cycloalkyl, wobei diese Reste einen C₁-C₄-Alkoxy, C₁-C₄-Alkylthio und/oder einen gegebenenfalls substituierten Phenylrest, wie insbesondere vorstehend genannt, tragen können;
      Phenyl, das durch einen oder mehrere, z.B. einen bis drei der folgenden Reste substituiert sein kann: Halogen, Nitro,
      Cyano, C₁-C₄-Alkyl, C₁-C₄-Halogenalkyl, C₁-C₄-Alkoxy, C₁-C₄-Halogenalkoxy oder C₁-C₄-Alkylthio, wobei diese Reste insbesondere den oben genannten entsprechen;
      oder R¹¹ und R¹² bilden gemeinsam eine C₃-C₁₂-Alkylenkette, welche ein bis drei C₁-C₄-Alkylgruppen tragen und ein Heteroatom aus der Gruppe Sauerstoff, Schwefel und Stickstoff enthalten kann, wie insbesondere bei R⁷ und R⁸ genannt.
g) R¹ ferner ein Rest 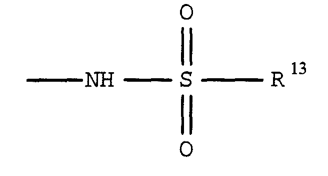 worin R¹³ bedeutet:
   C₁-C₄-Alkyl, C₃-C₆-Alkenyl, C₃-C₆-Alkinyl, C₃-C₈-Cycloalkyl wie insbesondere vorstehend genannt, wobei diese Reste einen C₁-C₄-Alkoxy-, C₁-C₄-Alkylthio- und/oder einen Phenylrest wie oben genannt tragen können;
   Phenyl, gegebenenfalls substituiert, insbesondere wie vorstehend genannt.
h) R¹ ein Rest 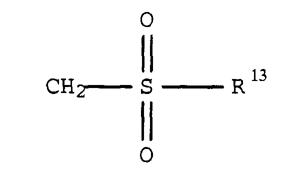 worin R¹³ die oben genannte Bedeutung hat.
   - R: kann weiterhin Tetrazol sein.

Im Hinblick auf die biologische Wirkung sind Carbonsäurederivate der allgemeinen Formel I - sowohl als reine Enantiomere bzw. reine Diastereomere oder als deren Mischung - bevorzugt, in denen die Substituenten folgende Bedeutung haben:
- R²: Wasserstoff, Hydroxy, N(C₁-C₄-Alkyl)₂, die bei R¹ im einzelnen genannten C₁-C₄-Alkyl-, C₁-C₄-Halogenalkyl-, C₁-C₄-Alkoxy-, C₁-C₄-Halogenalkoxy-, C₁-C₄-Alkylthiogruppen und Halogenatome, insbesondere Chlor, Methyl, Methoxy, Ethoxy, Difluormethoxy, Trifluormethoxy;
- X: Stickstoff oder CR¹⁴, worin
- R¹⁴: Wasserstoff oder Alkyl bedeutet oder CR¹⁴ zusammen mit CR³ einen 4- bis 5-gliedrigen Alkylen- oder Alkenylenring bildet, in der jeweils eine Methylengruppe durch Sauerstoff oder Schwefel ersetzt sein kann wie -CH₂-CH₂-O-, -CH=CH-O-, -CH₂-CH₂-CH₂-O-, -CH=CH-CH₂O-, insbesondere Wasserstoff, -CH₂-CH₂-O-, -CH(CH₃)-CH(CH₃)-O-, -C(CH₃)=C(CH₃)-O-, -CH=C(CH₃)-O- oder -C(CH₃)=C(CH₃)-S;
- R³: die bei R¹ genannten Wasserstoff, Hydroxy, N(C₁-C₄-Alkyl)₂, C₁-C₄-Alkyl-, C₁-C₄-Halogenalkyl-, C₁-C₄-Alkoxy-, C₁-C₄-Halogen-alkoxy-, C₁-C₄-Alkylthiogruppen und Halogenatome, insbesondere Chlor, Methyl, Methoxy, Ethoxy, Difluormethoxy, Trifluormethoxy oder mit R¹⁴ wie oben genannt zu einem 5-oder 6-gliedrigen Ring verknüpft ist;
- R⁴: und R⁵ Phenyl oder Naphthyl, die durch einen oder mehrere, z.B. einen bis drei der folgenden Reste substituiert sein können: Halogen, Nitro, Cyano, Hydroxy, Mercapto, Amino, C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylamino, Di-C₁-C₄-alkylamino, C₁-C₄-Alkylcarbonyl, C₁-C₄-Alkoxycarbonyl;
Phenyl oder Naphthyl, die orthoständig über eine direkte Bindung, eine Methylen-, Ethylen- oder Ethenylengruppe, ein Sauerstoff- oder Schwefelatom oder eine SO₂-, NH- oder N-Alkyl-Gruppe miteinander verbunden sind oder C₃-C₇-Cycloalkyl;
- R⁶: C₁-C₈-Alkyl, C₃-C₆-Alkenyl, C₃-C₆-Alkinyl oder C₃-C₈-Cycloalkyl wie insbesondere oben genannt, wobei diese Reste jeweils ein-oder mehrfach substituiert sein können durch: Halogen, Hydroxy, Nitro, Cyano, C₁-C₄-Alkoxy, C₃-C₆-Alkenyloxy, C₃-C₆-Alkinyloxy, C₁-C₄-Alkylthio, C₁-C₄-Halogenalkoxy, C₁-C₄-Alkylcarbonyl, Hydroxycarbonyl, C₁-C₄-Alkoxycarbonyl, C₁-C₄-Alkylamino, Di-C₁-C₄-alkylamino oder gegebenenfalls substituiertes Phenyl oder Phenoxy, wie insbesondere vorstehend genannt;
Phenyl oder Naphthyl, das durch einen oder mehreren der folgenden Reste substituiert sein kann: Halogen, Nitro, Cyano, Hydroxy, Amino, C₁-C₄-Alkyl, C₁-C₄-Halogenalkyl, C₁-C₄-Alkoxy, C₁-C₄-Halogenalkoxy, Phenoxy, C₁-C₄-Alkylthio, C₁-C₄-Akylamino oder C₁-C₄-Dialkylamino, wie insbesondere bei R⁷ und R⁴ genannt;
ein fünf- oder sechsgliedriger Heteroaromat, enthaltend ein bis drei Stickstoffatome und/oder ein Schwefel- oder Sauerstoffatom, welcher ein bis vier Halogenatome und/oder einen bis zwei der folgenden Reste tragen kann: C₁-C₄-Alkyl, C₁-C₄-Halogenalkyl, C₁-C₄-Alkoxy, C₁-C₄-Halogenalkoxy, C₁-C₄-Alkylthio, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio, wie insbesondere bei R⁴ genannt;
- Y: Sauerstoff;
- Z: Schwefel, Sauerstoff, -SO-, -SO₂-.

Besonders bevorzugt sind Verbindungen der Formel I - sowohl als reine Enantiomere bzw. reine Diastereomere oder als deren Mischung - in denen die Substituenten folgende Bedeutung haben:
- R²: C₁-C₄-Alkyl, C₁-C₄-Alkoxy
- X: Stickstoff oder CR¹⁴, worin
- R¹⁴: Wasserstoff oder Alkyl bedeutet oder CR¹⁴ zusammen mit CR³ einen 4- bis 5-gliedrigen Alkylen- oder Alkenylenring bildet wie z.B. -CH₂-CH₂-CH₂-, -CH=CH-CH₂-, in der jeweils eine Methylengruppe durch Sauerstoff oder Schwefel ersetzt sein kann wie -CH₂-CH₂-O-, -CH=CH-O-, -CH₂-CH₂-CH₂-O-, -CH=CH-CH₂O-, insbesondere Wasserstoff,
-CH₂-CH₂-O-, -CH(CH₃)-CH(CH₃)-O-, -C(CH₃)=C(CH₃)-O-,
-CH=C(CH₃)-O- oder -C(CH₃)=C(CH₃)-S;
- R³: die bei R¹ genannten C₁-C₄-Alkyl-, C₁-C₄-Alkoxy, C₁-C₄-Alkylthiogruppen oder mit R¹⁴ wie oben genannt zu einem 5- oder 6-gliedrigen Ring verknüpft ist;
- R⁴: und R⁵ Phenyl, die durch einen oder mehrere, z.B. einen bis drei der folgenden Reste substituiert sein können: Halogen, Nitro, Hydroxy, C₁-C₄-Alkyl, C₁-C₄-Alkoxy, C₁-C₄-Alkylthio oder
- R⁴: und R⁵ sind Phenylgruppen, die orthoständig über eine direkte Bindung, eine Methylen-, Ethylen- oder Ethenylengruppe, ein Sauerstoff- oder Schwefelatom oder eine SO₂-, NH- oder N-Alkyl-Gruppe miteinander verbunden sind; oder
- R⁴: und R⁵ sind C₃-C₇-Cycloalkyl;
- R⁶: C₁-C₈-Alkyl, C₃-C₆-Alkenyl oder C₃-C₈-Cycloalkyl, wobei diese Reste jeweils ein-oder mehrfach substituiert sein können durch: Halogen, Hydroxy, Nitro, Cyano, C₁-C₄-Alkoxy, C₃-C₆-Alkenyloxy, C₁-C₄-Alkylthio;
Phenyl oder Naphthyl, das durch einen oder mehreren der folgenden Reste substituiert sein kann: Halogen, Nitro, Cyano, Hydroxy, Amino, C₁-C₄-Alkyl, C₁-C₄-Halogenalkyl, C₁-C₄-Alkoxy, C₁-C₄-Halogenalkoxy, Phenoxy, C₁-C₄-Alkylthio, C₁-C₄-Akylamino oder C₁-C₄-Dialkylamino;
ein fünf- oder sechsgliedriger Heteroaromat, enthaltend ein Stickstoffatom und/oder ein Schwefel- oder Sauerstoffatom, welcher ein bis vier Halogenatome und/oder einen bis zwei der folgenden Reste tragen kann: C₁-C₄-Alkyl, C₁-C₄-Halogenalkyl, C₁-C₄-Alkoxy, C₁-C₄-Alkylthio, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy und/oder C₁-C₄-Alkylthio;
- Y: Sauerstoff;
- Z: Schwefel, Sauerstoff, -SO-, -SO₂-.

Die Verbindungen der vorliegenden Erfindung bieten ein neues therapeutisches Potential für die Behandlung von Hypertonie, pulmonalem Hochdruck, Myokardinfarkt, Angina Pectoris, akutem Nierenversagen, Niereninsuffizienz, zerebralen Vasospasmen, zerebraler Ischämie, Subarachnoidalblutungen, Migräne, Asthma, Atherosklerose, endotoxischem Schock, Endotoxin-induziertem Organversagen, intravaskulärer Koagulation, Restenose nach Angioplastie, benigne Prostata-Hyperplasie, ischämisches und durch Intoxikation verursachtes Nierenversagen bzw. Hypertonie.

Die gute Wirkung der Verbindungen läßt sich in folgenden Versuchen zeigen:

### Rezeptorbindungsstudien

Für Bindungsstudien wurden klonierte humane ET_{A}-Rezeptorexprimierende CHO-Zellen und Meerschweinchen-Kleinhirnmembranen mit > 60 % ETB- im Vergleich zu ET_{A}-Rezeptoren eingesetzt.

### Membranpräparation

Die ET_{A}-Rezeptor-exprimierenden CHO-Zellen wurden in F₁₂-Medium mit 10 % fötalem Kälberserum, 1 % Glutamin, 100 E/ml Penicillin und 0,2 % Streptomycin (Gibco BRL, Gaithersburg, MD, USA) vermehrt. Nach 48 h wurden die Zellen mit PBS gewaschen und mit 0,05 % trypsinhaltiger PBS 5 min inkubiert. Danach wurde mit F₁₂-Medium neutralisiert und die Zellen durch Zentrifugation bei 300 x g gesammelt. Zur Lyse der Zellen wurde kurz das Pellet mit Lysispuffer (5 mM Tris-HCl, pH 7,4 mit 10 % Glycerin) gewaschen und danach in einer Konzentration von 10⁷-Zellen/ml Lysispuffer 30 min bei 4°C inkubiert. Die Membranen wurden bei 20.000 x g 10 min zentrifugiert und das Pellet in flüssigem Stickstoff gelagert.

Meerschweinchenkleinhirne wurden im Potter-Elvejhem-Homogenisator homogenisiert und durch differentielle Zentrifugation 10 min bei 1.000 x g und wiederholte Zentrifugation des Überstandes 10 min bei 20.000 x g gewonnen.

### Bindungstests

Für den ET_{A}- und ET_{B}-Rezeptorbindungstest wurden die Membranen in Inkubationspuffer (50 mM Tris-HCl, pH 7,4 mit 5 mM MnCl₂, 40 µg/ml Bacitracin und 0,2 % BSA) in einer Konzentration von 50 µg Protein pro Testansatz suspendiert und bei 25°C mit 25 pM [125J]-ET₁ (ET_{A}-Rezeptortest) oder 25 pM [125J]-RZ₃ (ET_{B}-Rezeptortest) in Anwesenheit und Abwesenheit von Testsubstanz inkubiert. Die unspezifische Bindung wurde mit 10⁻⁷ M ET₁ bestimmt. Nach 30 min wurde der freie und der gebundene Radioligand durch Filtration über GF/B Glasfaserfilter (Whatman, England) an einem Skatron-Zellsammler (Skatron, Lier, Norwegen) getrennt und die Filter mit eiskaltem Tris-HCl-Puffer, pH 7,4 mit 0,2 % BSA gewaschen. Die auf den Filtern gesammelte Radioaktivität wurde mit einem Packard 2200 CA Flüssigkeitszintillationszähler quantifiziert.

### Funktionelles in vitro-Testsystem für die Suche nach Endothelinrezeptor (Subtyp A)-Antagonisten

Dieses Testsystem ist ein funktioneller, auf Zellen basierender Test für Endothelinrezeptoren. Bestimmte Zellen zeigen, wenn sie mit Endothelin 1 (ET1) stimuliert werden, einen Anstieg der intrazellulären Calciumkonzentration. Dieser Anstieg kann in intakten Zellen, die mit Calcium-sensitiven Farbstoffen beladen wurden, gemessen werden.

Aus Ratten isolierte 1-Fibroblasten, bei denen ein endogener Endothelinrezeptor vom A-Subtyp nachgewiesen wurde, wurden mit dem Fluoreszenzfarbstoff Fura 2-an wie folgt beladen: Nach Trypsinierung wurden die Zellen in Puffer A (120 mM NaCl, 5 mM KCl, 1,5 mM MgCl₂, 1 mM CaCl₂, 25 mM HEPES, 10 mM Glucose, pH 7,4) bis zu einer Dichte von 2 x 10⁶/ml resuspendiert und in 30 min bei 37°C im Dunkeln mit Fura 2-am (2 µM), Pluronics F-127 (0,04 %) und DMSO (0,2 %) inkubiert. Danach wurden die Zellen zweimal mit Puffer A gewaschen und zu 2 x 10⁶/ml resuspendiert.

Das Fluoreszenzsignal von 2 x 10⁵ Zellen pro ml bei Ex/Em 380/510 wurde bei 30°C kontinuierlich registriert. Zu den Zellen wurden die Testsubstanzen und nach einer Inkubationszeit von 3 min ET1 wurde die maximale Änderung der Fluoreszenz bestimmt. Die Antwort der Zellen auf ET1 ohne vorherige Zugabe einer Testsubstanz diente als Kontrolle und wurde gleich 100 % gesetzt.

### Testung der ET-Antagonisten in vivo

Männliche 250 - 300 g schwere SD-Ratten wurden mit Amobarbital narkotisiert, künstlich beatmet, vagotomisiert und despinalisiert. Die Arteria carotis und Vena jugularis wurden kathetisiert.

In Kontrolltieren führt die intravenöse Gabe von 1 µg/kg ET1 zu einem deutlichen Blutdruckanstieg, der über einen längeren Zeitraum anhält.

Den Testtieren wurde 5 min vor der ET1 Gabe die Testverbindungen i.v. injiziert (1 ml/kg). Zur Bestimmung der ET-antagonistischen Eigenschaften wurde der Blutdruckanstieg in den Testtieren mit dem in den Kontrolltieren verglichen.

### Endothelin-1 induzierter "sudden death" an Mäusen

Das Testprinzip besteht in der Hemmung des durch Endothelin verursachten plötzlichen Herztodes der Maus, der wahrscheinlich durch Verengung der Herzkranzgefäße bedingt ist, durch Vorbehandlung mit Endothelin-Rezeptorantagonisten. Nach intravenöser Injektion von 10 nmol/kg Endothelin im Volumen von 5 ml/kg Körpergewicht kommt es innerhalb weniger Minuten zum Tod der Tiere.

Die letale Endothelin-1 Dosis wird jeweils an einem kleinen Tierkollektiv überprüft. Wird die Prüfsubstanz intravenös appliziert, erfolgt meist 5 min danach die im Referenzkollektiv letale Endothelin-1 Injektion. Bei anderen Applikationsarten verlängern sich die Vorgabezeiten, gegebenenfalls bis zu mehreren Stunden.

Die Überlebensrate wird dokumentiert und effektive Dosen, die 50 % der Tiere 24 h oder länger gegen den Endothelin-Herztod schützen (ED 50) werden ermittelt.

### Funktioneller Gefäßtest für Endothelin-Rezeptorantagonisten

An Aortensegmenten des Kaninchens wird nach einer Vorspannung von 2 g und einer Relaxationszeit von 1 h in Krebs-Henseleitlösung bei 37°C und einem pH-Wert zwischen 7,3 und 7,4 zunächst eine K⁺-Kontraktur ausgelöst. Nach Auswaschen wird eine Endothelin-Dosiswirkungskurve bis zum Maximum erstellt.

Potentielle Endothelin-Antagonisten werden an anderen Präparaten des gleichen Gefäßes 15 min vor Beginn der Endothelin-Dosiswirkungskurve appliziert. Die Effekte des Endothelins werden in % der K⁺-Kontraktur berechnet. Bei wirksamen Endothelin-Antagonisten kommt es zur Rechtsverschiebung der Endothelin-Dosiswirkungskurve.

Die erfindungsgemäßen Verbindungen können in üblicher Weise oral oder parenteral (subkutan, intravenös, intramuskulär, intraperotoneal) verabfolgt werden. Die Applikation kann auch mit Dämpfen oder Sprays durch den Nasen-Rachenraum erfolgen.

Die Dosierung hängt vom Alter, Zustand und Gewicht des Patienten sowie von der Applikationsart ab. In der Regel beträgt die tägliche Wirkstoffdosis zwischen etwa 0,5 und 50 mg/kg Körpergewicht bei oraler Gabe und zwischen etwa 0,1 und 10 mg/kg Körpergewicht bei parenteraler Gabe.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al.: Pharmazeutische Technologie, Thieme-Verlag, Stuttgart, 1991). Die so erhaltenen Applikationsformen enthalten den Wirkstoff normalerweise in einer Menge von 0,1 bis 90 Gew.-%.

Synthesebeispiele

### Beispiel 1

### 2-Hydroxy-3-methoxy-3,3-diphenylpropionsäuremethylester

5 g (19,6 mmol) 3,3-Diphenyl-2,3-epoxypropionsäuremethylester wurden in 50 ml absolutem Methanol gelöst und bei 0°C mit 0,1 ml Bortrifluorid-Etherat versetzt. Man rührte 2 h bei 0°C und weitere 12 h bei Raumtemperatur. Das Lösungsmittel wurde abdestilliert, der Rückstand mit Essigester aufgenommen, mit Natriumhydrogencarbonat-Lösung und Wasser gewaschen und über Magnesiumsulfat getrocknet. Nach Abdestillieren des Lösungsmittels verblieben 5,5 g (88 %) eines schwach gelben Öls.

### Beispiel 2

### 2-Hydroxy-3-phenoxy-3,3-diphenylpropionsäuremethylester

5 g (19,6 mmol) 3,3-Diphenyl-2,3-epoxypropionsäuremethylester und 5,6 g (60 mmol) Phenol wurden zusammen 6 h auf 100°C erhitzt. Nach Abdestillieren des überschüssigen Phenols am Hochvakuum und chromatographischer Reinigung des Rückstands an Kieselgel mit Hexan/Essigestergemischen erhielt man 4,9 g (77 %) eines schwach gelben Öls.

### Beispiel 3

### 2-(4,6-Dimethoxy-pyrimidin-2-yloxy)-3-methoxy-3,3-diphenylpropionsäuremethylester

2,86 g (10 mmol) 2-Hydroxy-3-methoxy-3,3-diphenyl-propionsäuremethylester wurden in 40 ml Dimethylformamid gelöst und mit 0,3 g (12 mmol) Natriumhydrid versetzt. Man rührte 1 h und gab dann 2,2 g (10 mmol) 4,6-Dimethoxy-2-methylsulfonylpyrimidin zu. Nach 24 h Rühren bei Raumtemperatur wurde vorsichtig mit 10 ml Wasser hydrolysiert, mit Essigsäure ein pH-Wert von 5 eingestellt und das Lösungsmittel am Hochvakuum abdestilliert. Der Rückstand wurde in 100 ml Essigester aufgenommen, mit Wasser gewaschen, über Magnesiumsulfat getrocknet und das Lösungsmittel abdestilliert. Der Rückstand wurde mit 10 ml Ether versetzt und der entstandene Niederschlag abgesaugt. Nach dem Trocknen verblieben 3,48 g (82 %) eines weißen Pulvers.
Fp.: 81°C

### Beispiel 4

### 2-(4,6-Dimethoxy-pyrimidin-2-yloxy)-3-methoxy-3,3-diphenylpropionsäure

2,12 g (5 mmol) 2-(4,6-Dimethoxy-pyrimidin-2-yloxy)-3-methoxy-3,3-diphenyl-propionsäuremethylester wurden in 50 ml Dioxan gelöst, mit 10 ml 1 n KOH-Lösung versetzt und 3 h bei 100°C gerührt. Die Lösung wurde mit 300 ml Wasser verdünnt und mit Essigester zur Entfernung von nicht umgesetztem Ester extrahiert. Anschließend stellte man die Wasserphase mit verdünnter Salzsäure auf pH 1-2 und extrahierte mit Essigester. Nach Trocknen über Magnesiumsulfat und Abdestillieren des Lösungsmittels wurde der Rückstand mit einem Ether/Hexan-Gemisch versetzt und der gebildete Niederschlag abgesaugt. Nach dem Trocknen verblieben 1,85 g (90 %) eines weißen Pulver.
Fp.: 167°C

### Beispiel 5

### 2-(4,6-Dimethoxy-pyrimidin-2yloxy)-3-methoxy-3,3-diphenylnatriumpropionat

1,68 g (4mmol) 2-(4,6-Dimethoxy-pyrimidin-2-yloxy)-3-methoxy-3,3-diphenyl-propionsäure werden in 4 ml 1n NaOH + 100 ml Wasser gelöst. Die Lösung wird gefriergetrocknet und man erhält quantitativ das Natriumsalz der eingesetzten Carbonsäure.

### Beispiel 6

10 g (34,9 mmol) 2-Hydroxy-3-methoxy-3,3-diphenylpropionsäuremethylester wurden in je 50 ml Methanol und Eisessig gelöst, mit 1 ml RuO(OH)₂ in Dioxan versetzt und 30 h bei 100 bar und 100°C im Autoklaven mit H₂ hydriert. Der Katalysator wurde abfiltriert, der Ansatz eingeengt, mit Ether versetzt, mit NaCl-Lösung gewaschen, die organische Phase getrocknet und eingeengt. Man erhielt 10,1 g 3,3-Dicyclohexyl-2-hydroxy-3-methoxypropionsäuremethylester als Öl.

### Beispiel 7

### 2-Hydroxy-3-methoxy-3,3-diphenylpropionsäure

91,11 g (0,5 mol) Benzophenon und 45,92 g (0,85 mol) Natriummethylat wurden bei Raumtemperatur in 150 ml Methyl-tert. Butylether (MTB) suspendiert. Nach Abkühlen auf -10°C wurden 92,24 g (0,85 mol) Chloressigsäuremethylester so zugegeben, daß die Innentemperatur bis 40°C anstieg, wobei weiter mit einem Bad von -10°C gekühlt wurde. Anschließend wurde noch eine Stunde ohne Kühlung bei Eigentemperatur gerührt. Nach Zugabe von 250 ml Wasser und kurzem Rühren wurde die wäßrige Phase abgetrennt. Die MTB-Phase wurde mit 250 ml verdünnter Natriumchlorid-Lösung nachgewaschen. Nach Lösungsmitteltausch auf Methanol (250 ml) wurde eine Lösung von 1 g p-Toluolsulfonsäure in 10 ml Methanol bei Raumtemperatur zugegeben. Es wurde eine Stunde bei Eigentemperatur nachgerührt und dann auf Rückfluß erhitzt. Unter Abdestillieren des Methanols wurden 400 g einer 10 %igen Natronlauge zugetropft und abschließend 60 ml Wasser zugegeben. Das Methanol wurde solange abdestilliert, bis eine Sumpftemperatur von 97°C erreicht war. Nach Abkühlen auf 55°C wurde mit 190 ml MTB versetzt und mit ca. 77 ml konz. HCl bis pH 2 angesäuert. Nach Abkühlen auf Raumtemperatur wurde die wäßrige Phase abgetrennt und die organische Phase durch Abdestillieren von 60 ml MtB eingeengt. Durch Zugabe von 500 ml Heptan und langsamem Abkühlen auf Raumtemperatur wurde das Produkt auskristallisiert. Der grobkristalline Feststoff wurde abgesaugt, mit Heptan nachgewaschen und im Vakuumtrockenschrank bei 40°C bis zu Gewichtskonstanz getrocknet.
Ausbeute 108,9 g (80 %), HPLC > 99,5 Fl. %.

### Beispiel 8

### S-2-Hydroxy-3-methoxy-3,3-diphenylpropionsäure (Racematspaltung mit L-Prolinmethylester)

Zu 240 g einer 57 %igen methanolischen L-Prolinmethylester-Hydrochlorid-Lösung (0,826 mol) wurden bei Raumtemperatur 148,8 g einer 30 %igen methanolischen Natriummethanolat-Lösung (0,826 mol) zugetropft, 2,4 1 MTB und 225 g (0,826 mol) 2-Hydroxy-3-methoxy-3,3-diphenylpropionsäure zugegeben. Nach Abdestillieren von 2680 ml MTB-Methanol-Gemisch und gleichzeitigem Zutropfen von 2,4 1 MTB wurde langsam auf Raumtemperatur abgekühlt, das Kristallisat (R-2-Hydroxy-3-methoxy-3,3-diphenylpropionsäure x L-Prolinmethylester) abgesaugt und der Feststoff mit 150 ml MTB nachgewaschen. Das Filtrat wurde durch Abdestillieren von 1,5 1 MTB eingeengt und mit 1,0 1 Wasser versetzt. Bei Raumtemperatur wurde mit konz. Salzsäure auf pH 1,2 eingestellt, nach Rühren und Phasentrennung die wäßrige Phase abgetrennt und mit 0,4 1 MTB nachextrahiert. Die vereinigten organischen Phasen wurden mit 0,4 1 Wasser extrahiert. Nach Abziehen des MTB wurde der Rückstand in 650 ml Toluol unter Rückfluß gelöst und das Produkt durch Animpfen und langsames Abkühlen kristalliert. Nach Absaugen, Nachwaschen mit Toluol und Trocknen im Vakuumtrockenschrank wurden 78,7 g S-2-Hydroxy-3-methoxy-3,3-diphenylpropionsäure erhalten (Ausbeute 35 % bezogen auf das Racemat).
Chirale HPLC: 100 %ig

### HPLC: 99,8 %

### Beispiel 9

### S-2-Hydroxy-3-methoxy-3,3-diphenylpropionsäure (Racematspaltung mit (S)-1- (4-Nitrophenyl)ethylamin)

100 g (0,368 mol) 2-Hydroxy-3-methoxy-3,3-diphenylpropionsäure wurden in 750 ml Aceton und 750 ml MTB unter Rückfluß mit 30,5 g (0,184 mol) (S)-1-(4-Nitrophenyl)ethylamin versetzt, angeimpft, eine Stunde unter Rückfluß gekocht und unter Kristallisation langsam auf Raumtemperatur abgekühlt. Das Kristallisat (S-2-Hydroxy-3-methoxy-3,3-diphenylpropionsäure x (S)-1-(4-Nitrophenyl)ethylamin) wurde abgesaugt und mit MTB nachgewaschen. Nach Suspendieren des Rückstands in 500 ml Wasser und 350 ml MTB wurde bei Raumtemperatur mit konz. Salzsäure auf pH 1,2 eingestellt, nach Rühren und Phasentrennung die wäßrige Phase abgetrennt und mit 150 ml MTB nachextrahiert. Die vereinigten organische Phasen wurden mit 100 ml Wasser extrahiert. Nach Abdestillieren von 370 ml MTB wurde unter Rückfluß mit 390 ml n-Heptan versetzt und unter Kristallisation des Produkts langsam auf Raumtemperatur abgekühlt. Nach Absaugen, Nachwaschen mit n-Heptan und Trocknen im Vakuumtrockenschrank wurden 35,0 g S-2-Hydroxy-3-methoxy-3,3-diphenylpropionsäure erhalten (Ausbeute 35 % bezogen auf das Racemat).
Chirale HPLC: 100 %ig
HPLC: 99,8 %

### Beispiel 10

### 3-Methoxy-2-(4-methoxy-6,7-dihydro-5H-cyclopentapyrimidin-2-yloxy)-3,3-diphenyl-propionsäurebenzylester

24,48 g (90 mmol) 3-Methoxy-3,3-diphenyl-2-hydroxypropionsäure wurden in 150 ml DMF gelöst und mit 13,7 g (99 mmol) Kaliumcarbonat versetzt. Die Suspension wurde 30 min. bei Raumtemperatur gerührt. Anschließend wurden über 5 min. 10,7 ml (90 mmol) Benzylbromid zugetropft und 1 h nachgerührt, wobei die Temperatur auf 32°C anstieg.

Zu diesem Ansatz gab man nacheinander 24,84 g (180 mmol) K₂CO₃ und 20,52 g (90 mmol) 2-Methansulfonyl-4-methoxy-6,7-dihydro-5H-cyclopentapyridin und rührte 3 h bei 80°C.

Zur Aufarbeitung verdünnte man den Kolbeninhalt mit ca. 600 ml H₂O, säuerte vorsichtig mit konz. HCl an und gab 250 ml Essigester hinzu. Es fielen 31,4 g sauberes Produkt aus, das abfiltriert wurde.

Aus der Mutterlauge wurde die Essigesterphase abgetrennt, die wäßrige Phase nochmals mit Essigester extrahiert und die vereinigten organischen Phasen eingeengt. Der ölige Rückstand (19 g) wurde chromatographisch gereinigt (Cyclohexan/Essigester = 9/1) und man erhielt weitere 10,5 g sauberes Produkt.

Gesamtausbeute: 41,9 g (82,2 mmol) =̂ 91 %
Fp.: 143-147°C
MS: MH⁺ = 511

### Beispiel 11

### 3-Methoxy-2-(4-methoxy-(6,7-dihydro-5H-cyclopentapyrimidin-2-yloxy)-3,3-diphenylpropionsäure

40 g (78,4 mmol) 3-Methoxy-2-(4-methoxy-6,7-dihydro-5H-cyclopentapyrimidin-2-yloxy)-3,3-diphenyl-propionsäurebenzylester wurden in 400 ml Essigester/Methanol (4:1) gelöst, mit ca. 500 mg Palladium auf Aktivkohle (10 %ig) versetzt und einer Wasserstoffatmosphäre ausgesetzt, bis keine Gasaufnahme mehr stattfand. Der Katalysator wurde abfiltriert, die Lösung eingedampft und der Rückstand aus Ether auskristallisiert.

### Beispiel 12

### 2S-3,3-Diphenyl-oxiran-2-carbonsäureethylester

2,57 g (10,2 mmol) 3,3-Diphenyl-acrylsäureethylester und 464 mg 4-Phenylpyridin-N-oxid wurden in 24 ml Methylenchlorid gelöst und mit 432 mg (6,5 mol %) (5,5)-(+)-N,N'-Bis(3,5-di-tert.-butylsalicyliden)-1,2-cyclohexandiamino-mangan(III)chlorid versetzt. Unter Eiskühlung gab man 6,4 ml einer 12 %igen Natriumhypochlorid-Lösung hinzu, rührt 30 Minuten bei Eiskühlung und über Nacht bei Raumtemperatur. Die Reaktionslösung wurde mit Wasser auf 200 ml verdünnt, mit Ether extrahiert, getrocknet und eingedampft. Man erhielt 2,85 g eines farblosen Öls. Nach Reinigung mittels NPLC (Cyclohexan:Essigester = 9:1) erhielt man 1,12 g Öl mit einem Enantiomerenverhältnis von ca. 8:1 zugunsten der S-Konfiguration.

¹H-NMR [CDCl₃],
δ = 1,0 (tr, 3H); 3,9 (m, 3H); 7,3 (m, 10H)

### Beispiel 13

### 2-Methylsulfonyl-6,7-dihydro-5H-cyclopentapyrimidin-4-ol

Zu 29,6 g (528 mmol) KOH in 396 ml Methanol gab man nacheinander 46,9 g (330 mmol) Cyclopentanon-2-carbonsäuremethylester und 53,5 g (192 mmol) 5-Methylisothioharnstoff-Sulfat und rührte über Nacht bei Raumtemperatur. Das Reaktionsgemisch wurde mit 1n Salzsäure angesäuert und mit Wasser verdünnt. Die ausgefallenen Kristalle wurden abgesaugt und getrocknet. Man erhielt 20 g Kristalle.

### Beispiel 14

### 4-Chloro-2-methylsulfanyl-6,7-dihydro-5H-cyclopentapyrimidin

Zu 20 g (110 mmol) gab man 255 ml Phosphoroxychlorid und rührt 3 Stunden bei 80°C. Phosphoroxychlorid wurde abgedampft, der Rückstand mit Eis zersetzt und die ausgefallenen Kristalle abgesaugt. Man erhielt 18,5 g eines bräunlichen Feststoffs.

### Beispiel 15

### 4-Methoxy-2-methylsulfonyl-6,7-dihydro-5H-cyclopentapyrimidin

18,05 g (90 mmol) 4-Chloro-2-methylsulfonyl-6,7-dihydro-5H-cyclopentapyrimidin wurden in 200 ml Methanol gelöst. Bei 45°C werden 16,7 g Natriummethylat (als 30 %ige Lösungen in Methanol) zugetropft und 2 Stunden nachgerührt. Die Reaktionslösung wurde eingedampft, in Essigester aufgenommen, mit verdünnter Salzsäure angesäuert und der Essigester-Extrakt eingedampft. Es blieben 15,5 g eines Öls zurück.

¹H-NMR [DMSO],
δ = 2,1 (quintett, 2H); 2,5 (s, 3H) ;
2,8 (dtr, 4H); 3,9 (s, 3H) ppm

### Beispiel 16

### 2-Methylsulfonyl-4-methoxy-6,7-dihydro-5H-cyclopentopyrimidin

15 g (76,2 mmol) 4-Methoxy-2-methylsulfonyl-6,7-dihydro-5H-cyclopentapyrimidin wurden in 160 ml Eisessig/Methylenchlorid (1:1) gelöst und mit 1,3 g Natriumwolframat versetzt. Bei 35°C tropfte man 17,5 ml (170 ml) einer 30 %igen H₂O₂-Lösung zu. Anschließend wurde mit 500 ml Wasser und 100 ml Methylenchlorid verdünnt, die organische Phase abgetrennt, getrocknet und eingedampft. Es blieben 14 g Öl zurück, das aus Ether auskristallisiert.

¹H-NMR [CDCl₃],
δ = 2,2 (quintett, 2H); 3,0 (dtr., 4H);
3,3 (s, 3H); 4,1 (s, 3H) ppm

### Beispiel 17

### 1-Benzolsulfonyl-3-(4,6-dimethoxypyrimidin-2-yloxy)-4-methoxy-4,4-diphenyl-butan-2-on

0,37 g (2,4 mmol) Phenylmethansulfon wurden in 10 ml trockenem THF gelöst; anschließend bei -70°C 2 eq. Butyllithium (2,94 ml; 1,6 molare Lösung in Hexan) zugetropft. Nach 1 h bei -70°C wurde 1 g (2,4 mmol) 2-(4,6-Dimethoxypyrimidin-2-yloxy)-3-methoxy-3,3-diphenylpropinsäuremethylester - gelöst in 5 ml THF - zugetropft.

Das Reaktionsgemisch wurde 1 h bei -70°C, 1 h bei -10°C nachgerührt und dann auf Raumtemperatur erwärmt.

Zur Aufarbeitung tropfte man ca. 10 ml gesättigte NH₄Cl-Lösung zu, extrahierte gründlich mit Ethylacetat, die vereinigten org. Phasen mit gesättigter N-Cl-Lösung und trocknete über Na₂SO₄.
Der nach Trocknen und Einengen erhaltene Rückstand wurde über Chromatographie an Kieselgel (n-Heptan/Ethylacetat 15 % → 30 %) und anschließend MPLC an RP-Kieselgel (Acetonitril/H₂O + TFA) gereinigt; als Produkt wurden 0,3 g eines weißen amorphen Pulvers erhalten.

### Beispiel 18

### 3,3-Diphenyl-oxiram-2-carbonitril

3,1 g (54,9 mmol) Natriummethylat wurden in 20 ml trockenem THF suspendiert, und anschließend bei -10°C eine Mischung aus 5 g (27,4 mmol) Benzophenon und 4,2 g (54,9 mmol) Chloracetonitril zugetropft.

Das Reaktionsgemisch wurde ca. 2 h bei -10°C nachgerührt, anschließend auf Wasser gegossen und mit Ethylacetat mehrmals extrahiert. Die vereinigten organischen Phasen wurden über Na₂SO₄ getrocknet, eingeengt, und der verbleibende Rückstand durch Chromatographie an Kieselgel (n-Heptan/Ethylacetat) gereinigt. Ausbeute: 1,2 g (20 %)

¹H-NMR [CDCl₃],
δ = 3,9 (s, 1H); 7,4-7,5 (m, 10 H) ppm

### Beispiel 19

### 2-Hydroxy-3-methoxy-3,3-diphenyl-propionitril

6,5 (29,4 mmol) 3,3-Diphenyl-oxiran-2-carbonitril wurden in 60 ml Methanol gelöst und bei 0°C mit ca. 2 ml Bortrifluorid-Etherat-Lösung versetzt. Das Reaktionsgemisch wurde erneut 1 h bei 0°C, dann bei Raumtemperatur über Nacht nachgerührt. Zur Aufarbeitung wurde mit Diethylether verdünnt, mit gesättigter NaCl-Lösung gewaschen, die organische Phase über Na₂SO₄ getrocknet und eingeengt. Als Rückstand verblieben 7,3 g eines weißen amorphen Pulvers, das in den weiteren Umsetzungen direkt eingesetzt wurde.

¹H-NMR [CDCl₃],
δ = 2,95 (breites s, OH), 3,15 (s, 3H),
5,3 (s, 1H), 7,3-7,5 (m, 10) ppm

### Beispiel 20

### 2-(4,6-Dimethoxy-pyrimidin-2-yloxy)-3-methoxy-3,3-diphenylpropionitril

7,3 g (28,8 mmol) 2-Hydroxy-3-methoxy-3,3-diphenylpropionitril wurden in 90 ml DMF gelöst, und mit 4 g (28,8 mmol) K₂CO₃ und 6,3 g (28,8 mmol) 2-Methansulfonyl-4,6-dimethoxy-pyrimidin versetzt. Die Mischung wurde für ca. 12 h bei Raumtemperatur gerührt, anschließend auf Wasser gegossen und mit Ethylacetat extrahiert. Die vereinigten org. Phasen wurden erneut mit H₂O gewaschen, getrocknet und eingeengt. Der so erhaltene Rückstand wurde dann durch Chromatographie an Kieselgel (n-Heptan/Ethylacetat) gereinigt.
Ausbeute: 6,9 g weißes amorphes Pulver

FAB-MS: 392 (M+H⁺)
¹H-NMR [CDCl₃],
δ = 3,3 (s, 3H); 4,95 (s, 6H), 5,85 (s, 1H) ;
6,3 (s, 1H); 7,3-7,5 (m, 10H) ppm

### Beispiel 21

### 5-[2-(4,6-Dimethoxy-pyrimidin-2-yloxy)-3-methoxy-3,3-diphenyl)-propyl]-1H-tetrazol

0,5 g (1,3 mmol) Nitril wurden in 10 ml Toluol gelöst, nacheinander 85 mg (1,3 mmol) NaN₃ und 460 mg (1,4 mmol) Bu₃SnCl zugesetzt und anschließend die Mischung für ca. 40 h auf Rückfluß erhitzt. Nach dem Abkühlen wurde mit Ethylacetat verdünnt, mit 10 % wäßriger KF-Lösung und mit NaCl-Lösung gewaschen. Nach Trocknen über MgSO₄ und Einengen verblieben 1,0 g eines gelben Öls, das durch Chromatographie an Kieselgel (n-Heptan/Ethylacetat) gereinigt wurde.

Nach Einengen der Fraktionen wurden 60 mg des 1H-Tetrazols und 110 mg des 1-Methyl-Tetrazols - jeweils als amorphe weiße - Feststoffe erhalten.

### 5-[2-(4,6-Dimethoxy-pyrimidin-2-yloxy)-3-methoxy-3,3-diphenyl)-propyl]-1H-tetrazol

Elektrospray-MS: 435 (M+H⁺)
¹H-NMR (CDCl₃):
δ (ppm) 3,28 (s, 3H), 3,85 (s, 6H), 5,75 (s, 1H), 7,25-7,40
(m, 10H), 7, 50 (s, 1H) .

### 5-[2-(4,6-Dimethoxy-pyrimidin-2-yloxy)-3-methoxy-3,3-diphenyl)-propyl]-1-methyl-tetrazol

Elektrospray-MS; 471 (M+H⁺)
¹H-MMR (CDCl₃):
δ (ppm) 3,0 (s, 3H), 3,35 (s, 3H9, 3,80 (s, 6H), 5,75 (s, 1H),
7,30-7,40 (m,11H).

### Beispiel 22

### 2-(4,6-Dimethoxy-pyrimidin-2-yloxy)-3-methylsulfinyl-3,3-diphenyl-propionsäure

1,2 g (2,9 mmol) 2-(4,6-Dimethoxy-pyrimidin-2-yloxy)-3-methylsulfonyl-3,3-diphenyl-propionsäure wurden in 15 ml Eisessig bei 0°C vorgelegt und 294 µl 30 %iges H₂O₂ zugetropft. Man ließ über Nacht bei Raumtemperatur rühren, goß auf Wasser, extrahierte mit CH₂Cl₂ und wusch mit Natriumthiosulfat-Lösung und Kochsalz-Lösung. Nach dem Trocknen isolierte man 1 g Substanz als weißen Schaum.

### Beispiel 23

### 2-(4,6-Dimethoxy-pyrimidin-2-yloxy)-3-methylsulfonyl-3,3-diphenyl-propionsäure

0,6 g (1,45 mmol) 2-(4,6-Dimethoxy-pyrimidin-2-yloxy)-3-methylsulfonyl-3,3-diphenyl-propionsäure wurden in 15 ml Eisessig bei Raumtemperatur vorgelegt und 294 µl 30 %iges H₂O₂ zugetropft. Man ließ über Nacht bei Raumtemperatur rühren, erhitzte weitere 3 h auf 50°C, goß auf Wasser und wusch mit Natriumthiosulfat-Lösung und Kochsalz-Lösung. Nach dem Trocknen isolierte man 400 mg als weißen Feststoff.

Analog lassen sich die in Tabelle 1 aufgeführten Verbindungen herstellen.

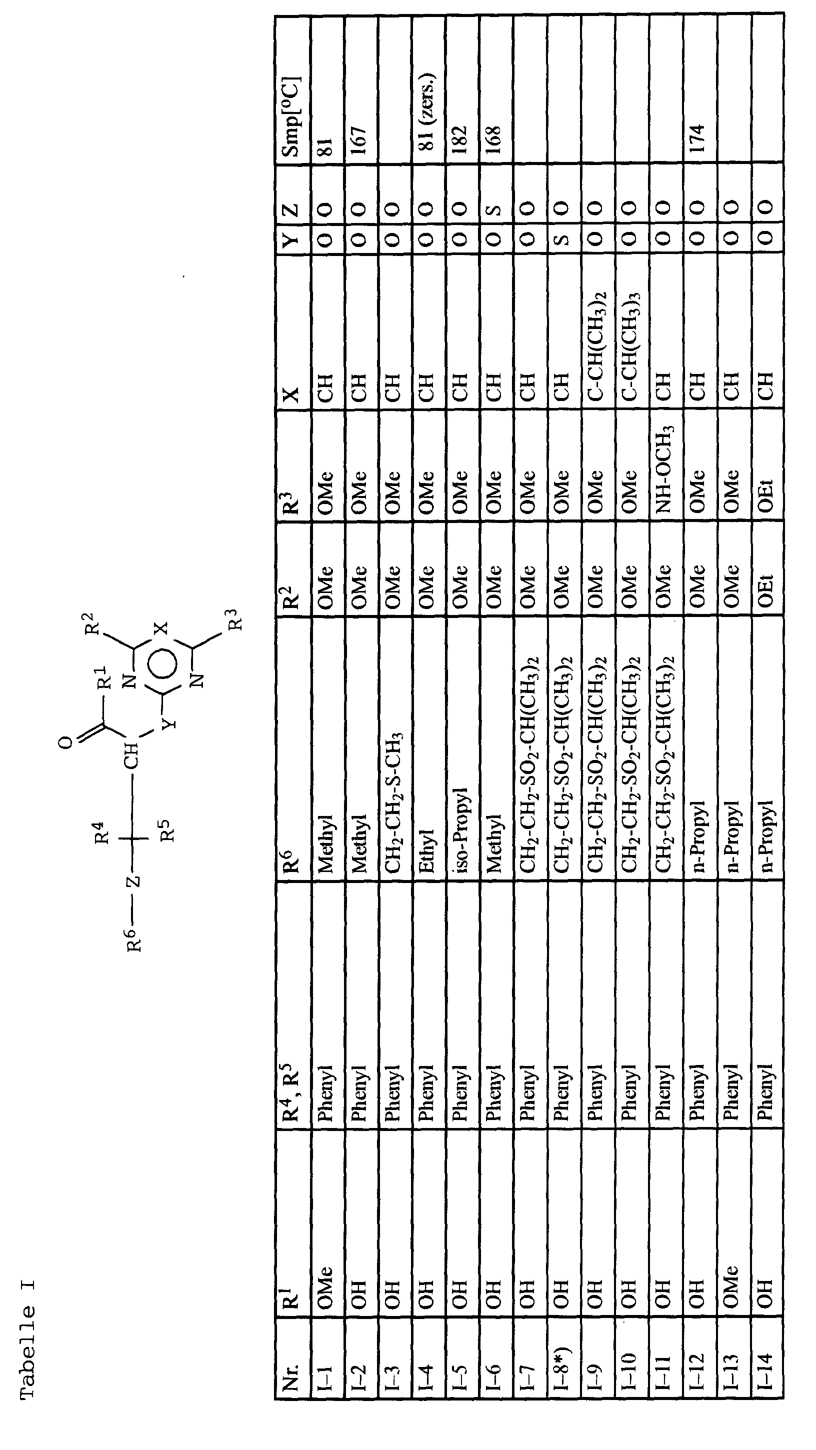

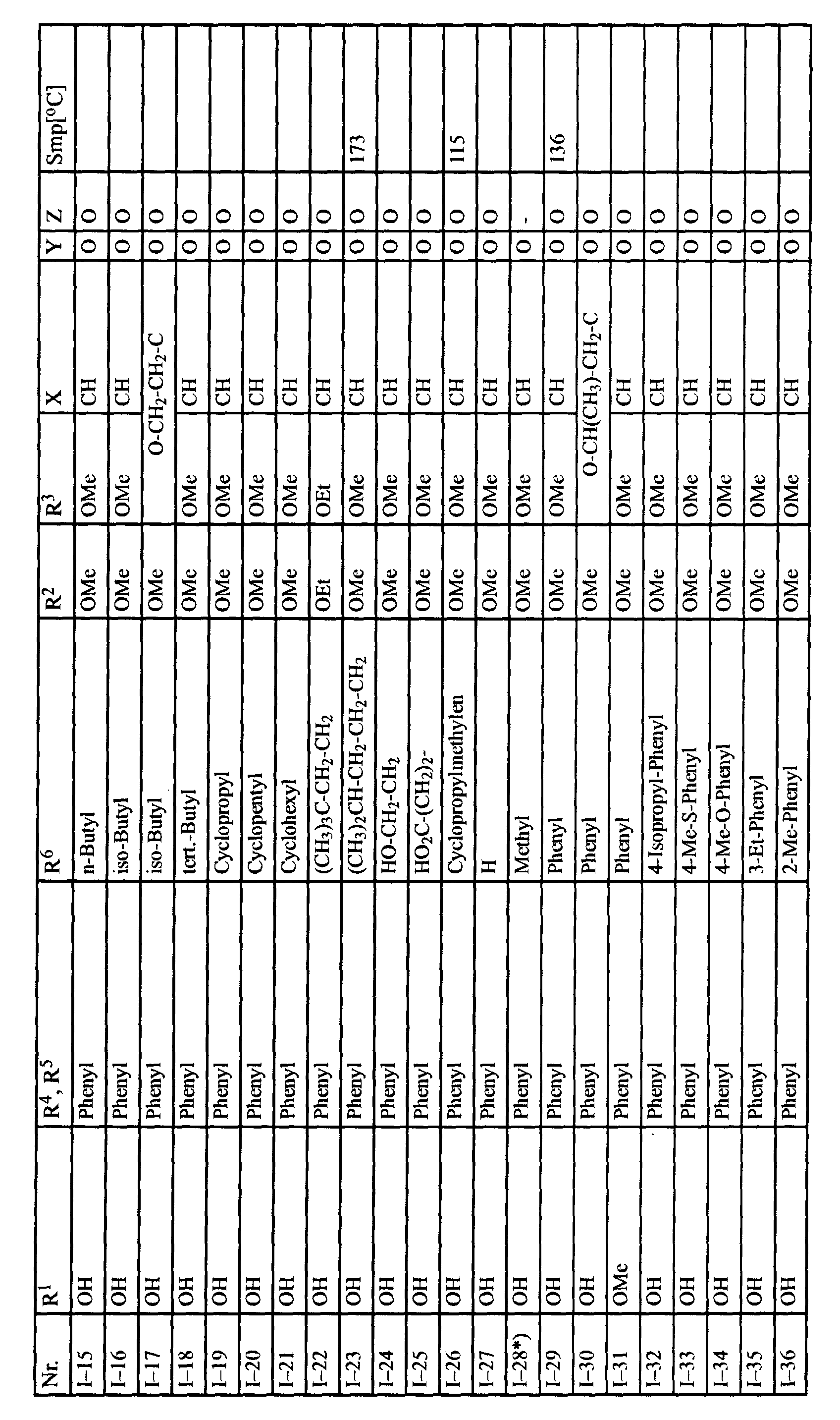

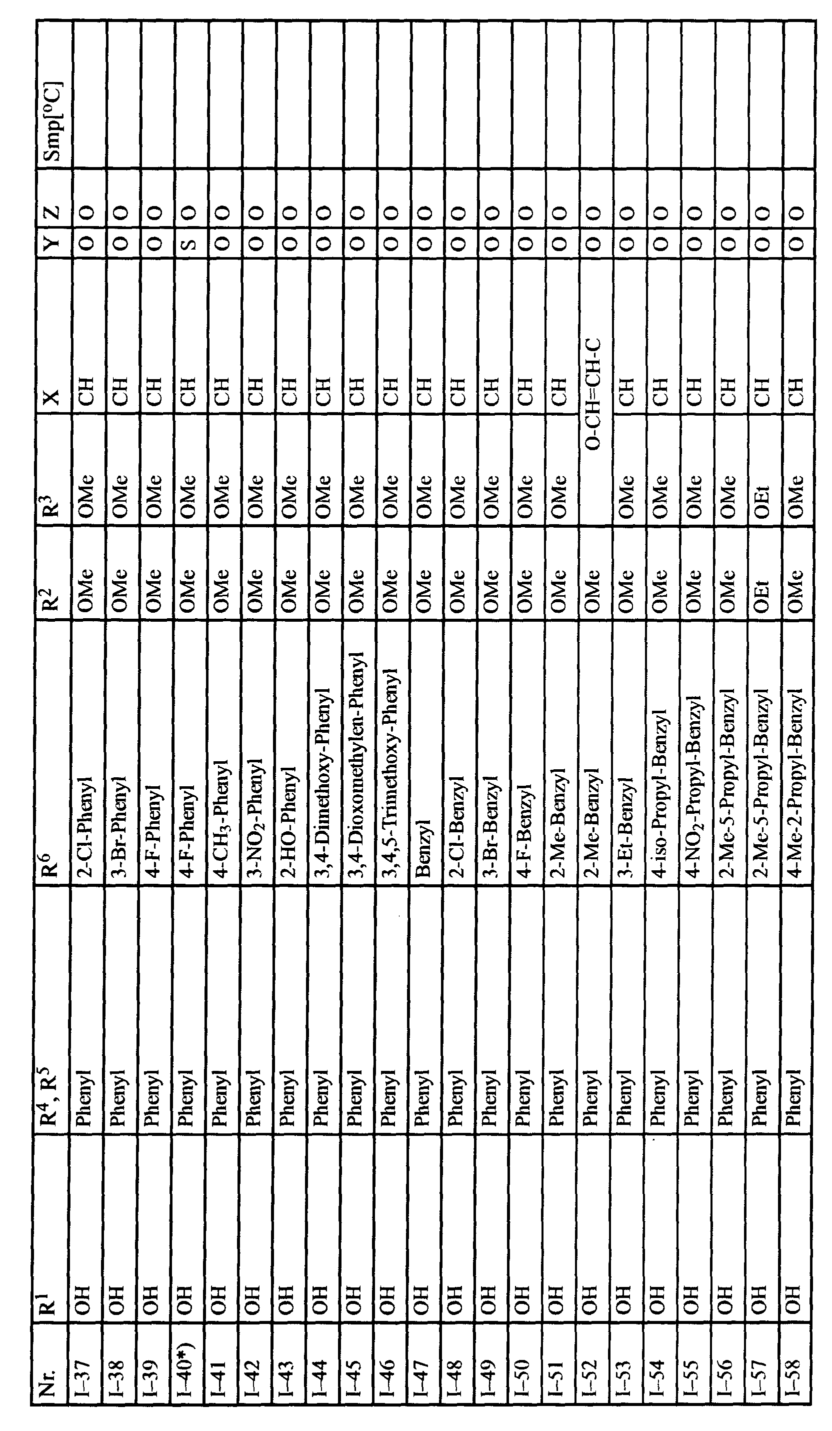

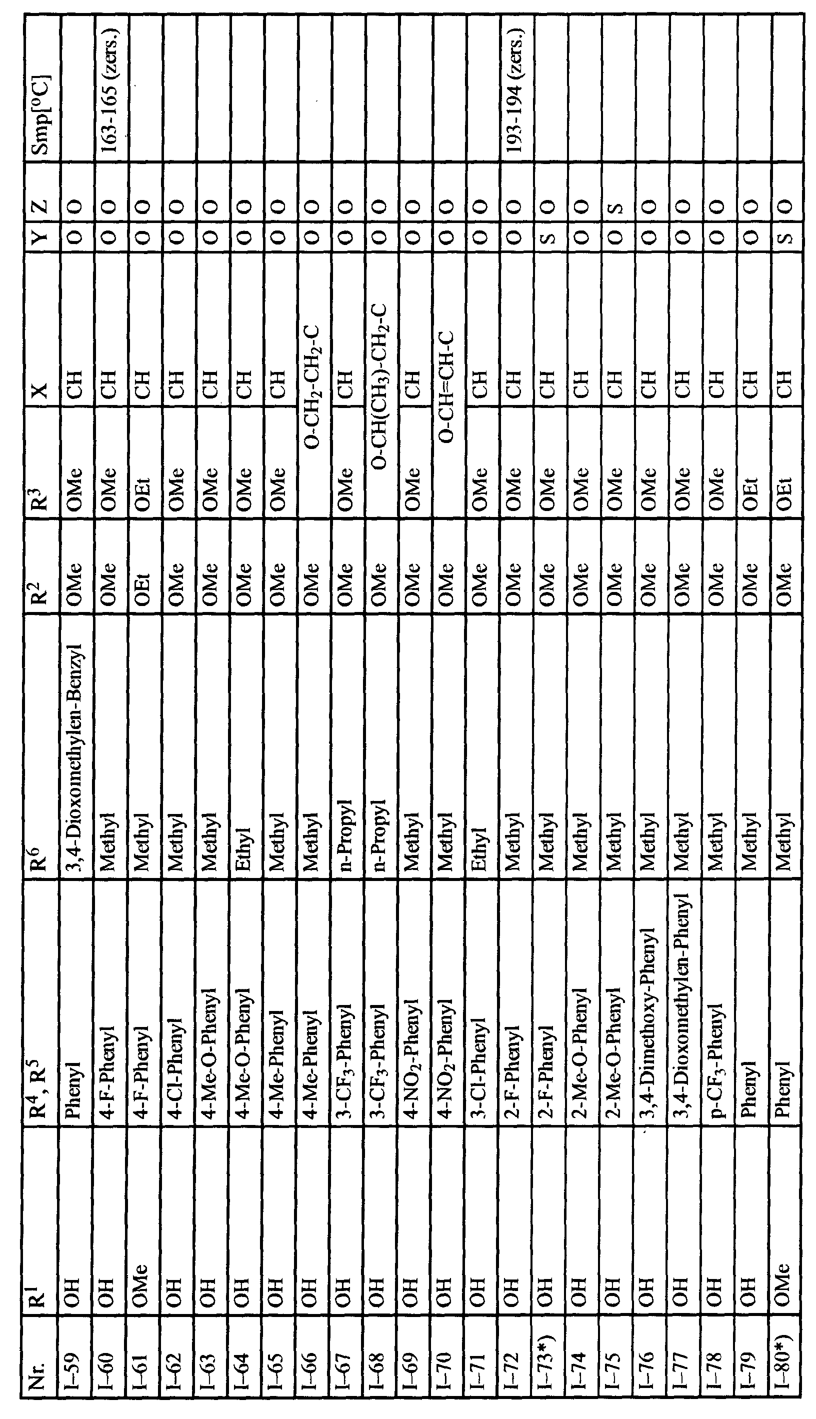

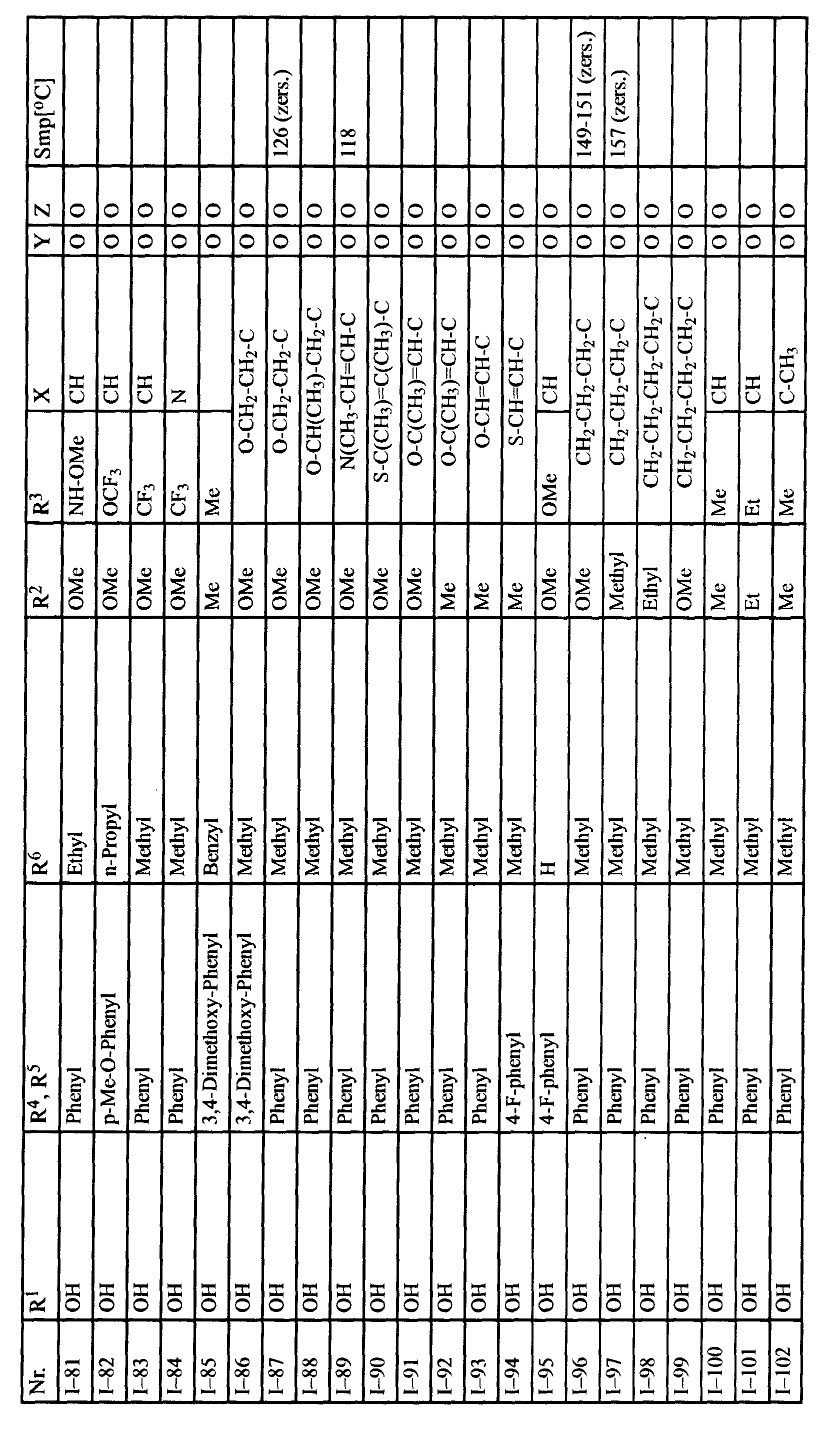

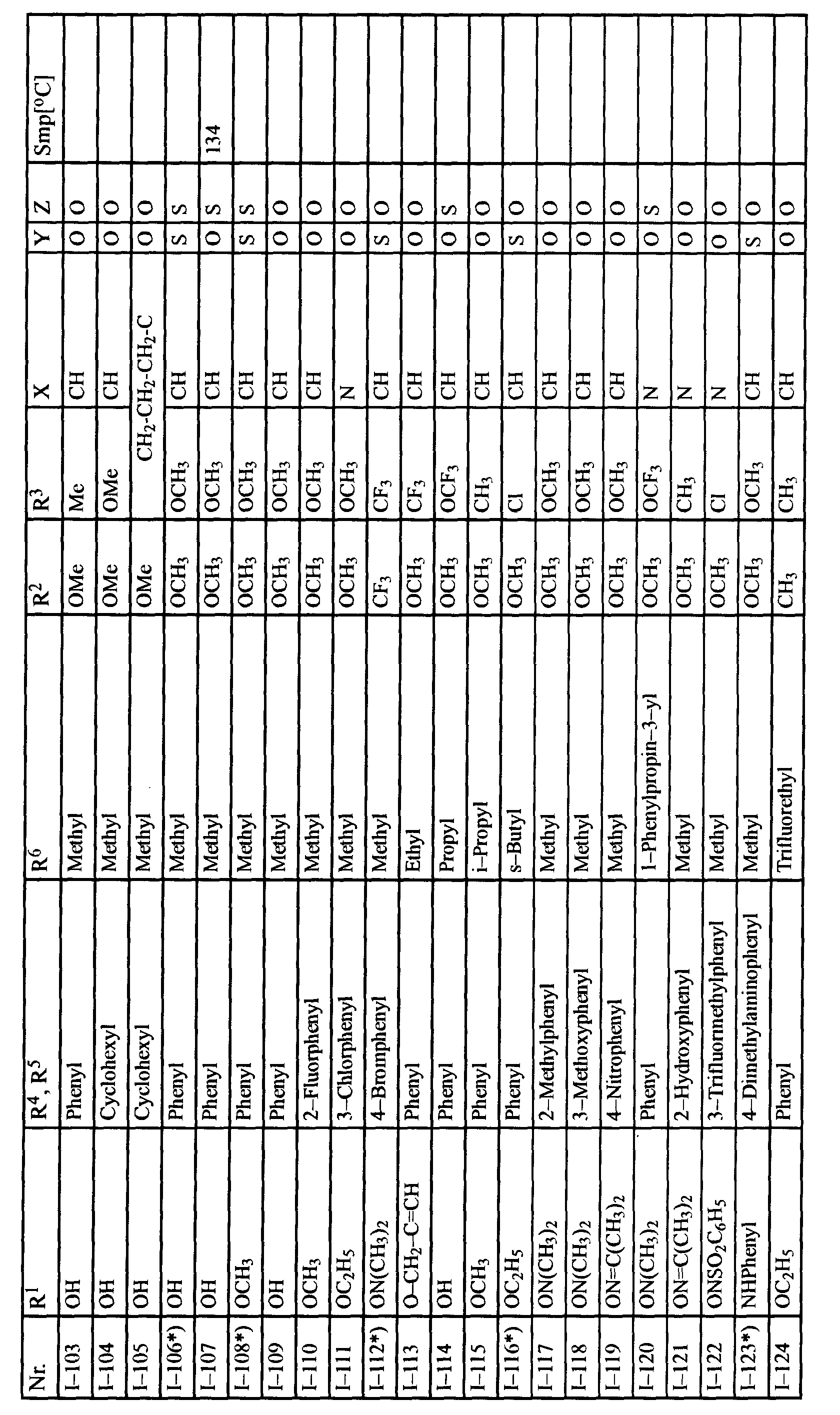

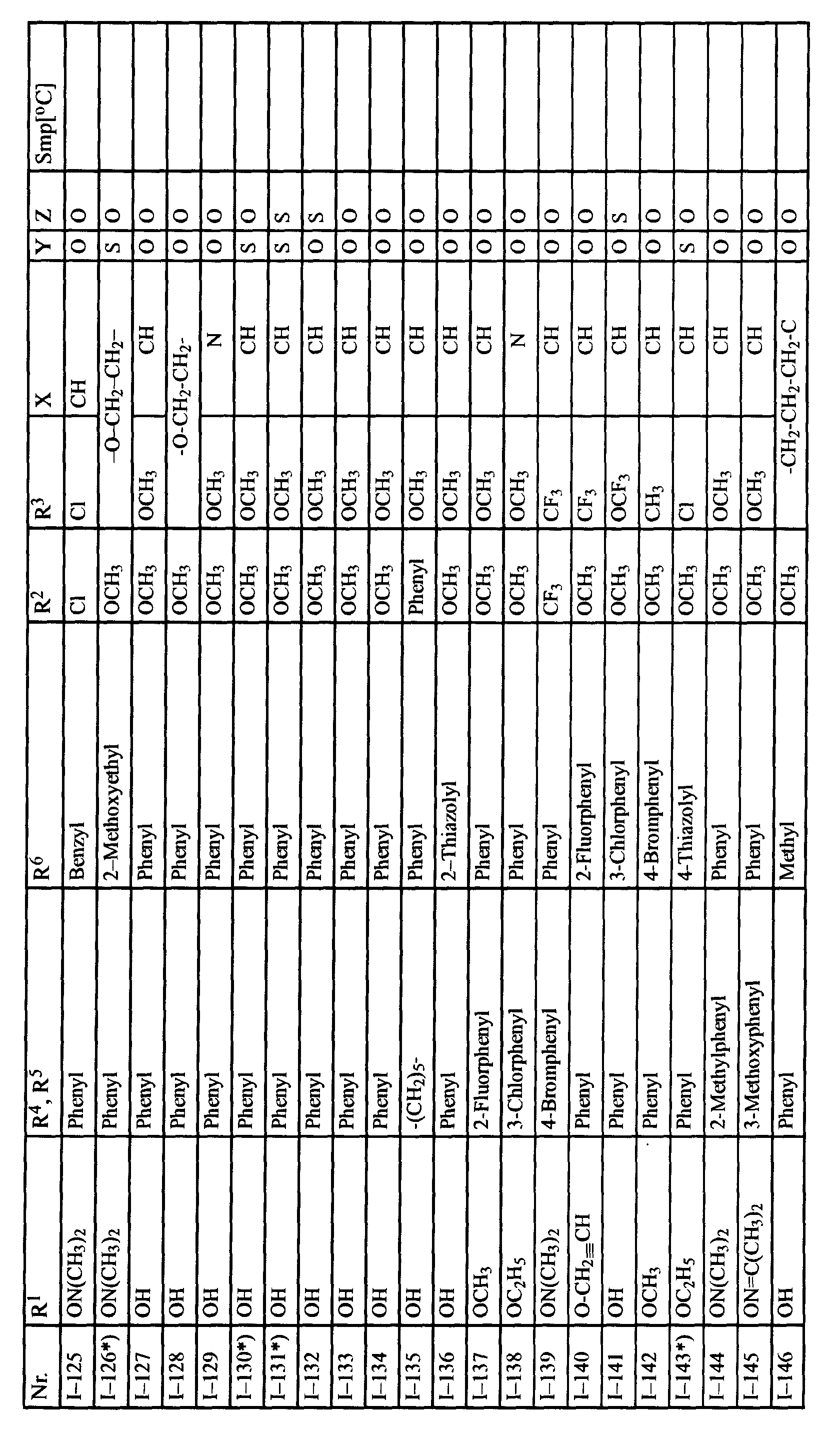

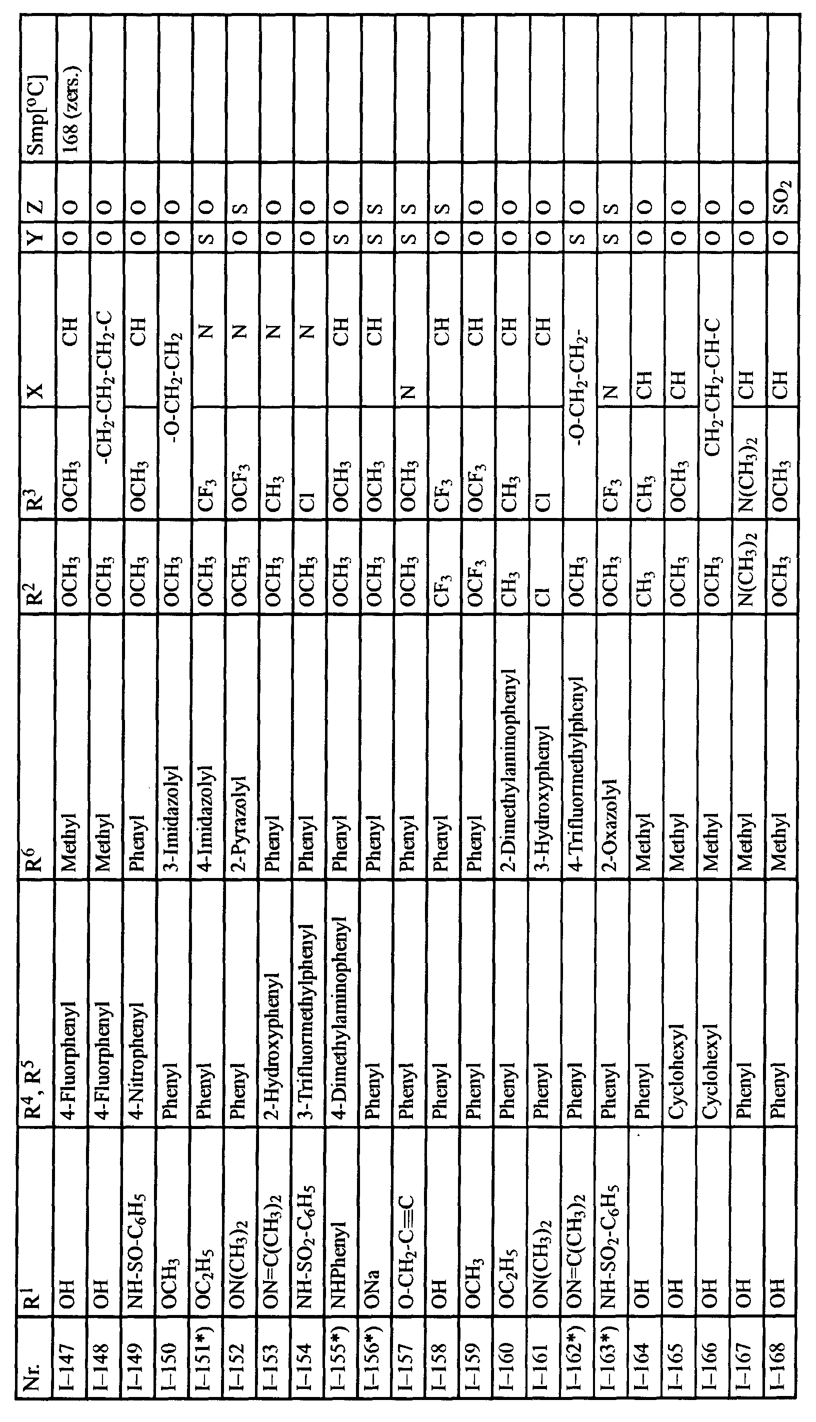

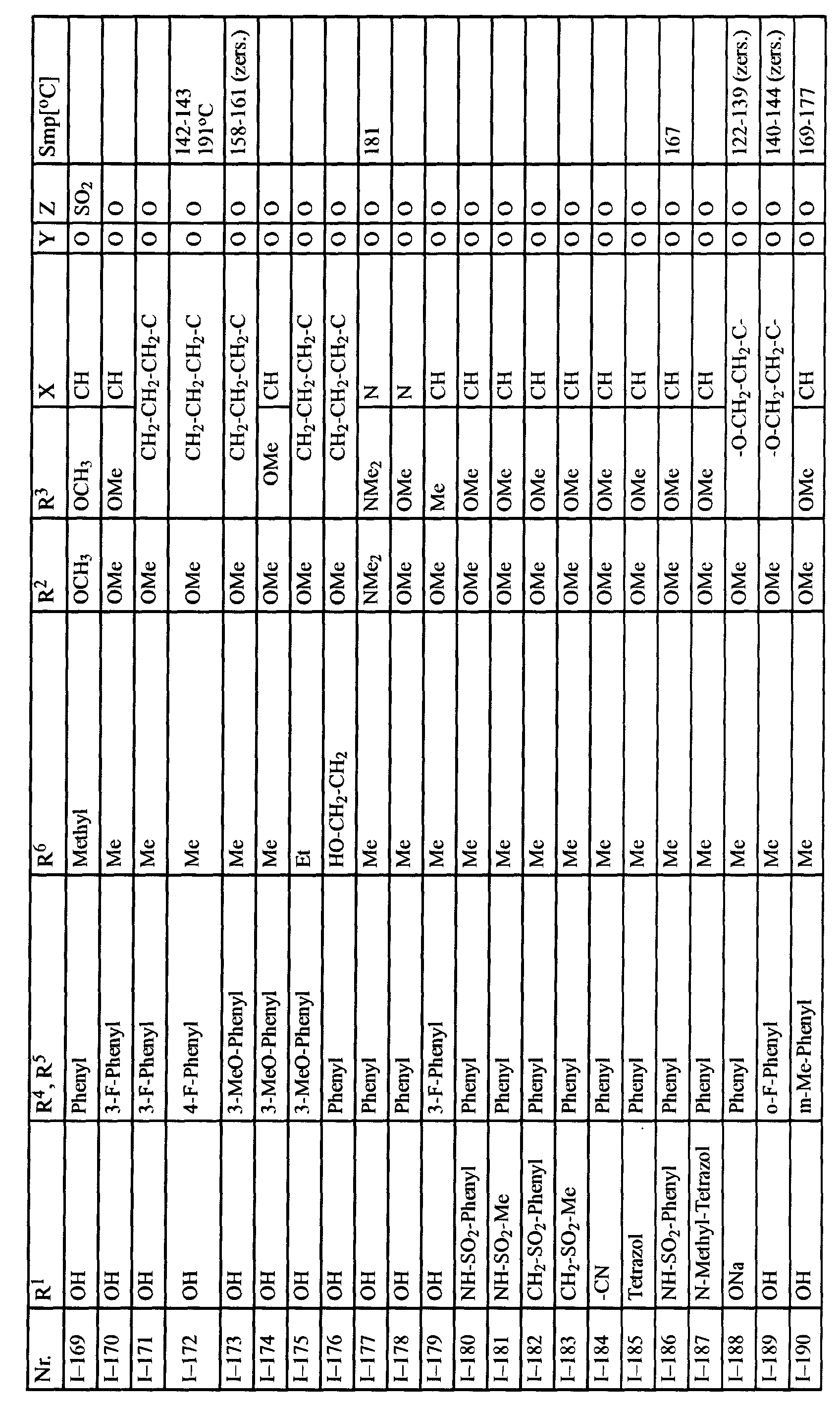

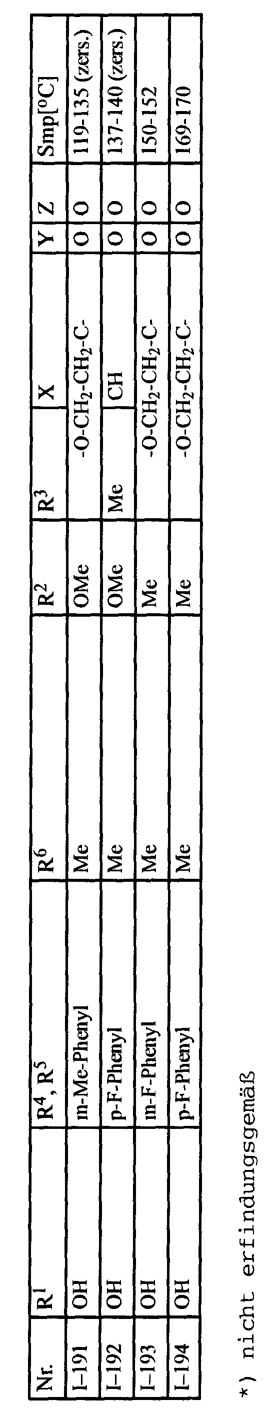

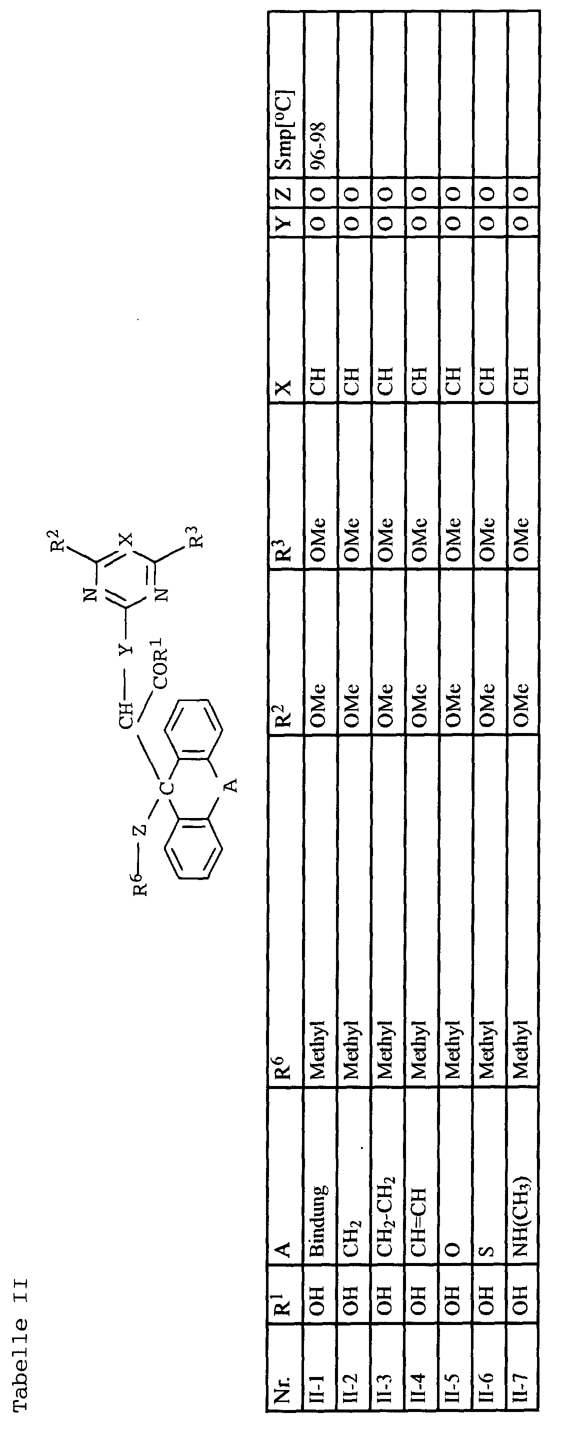

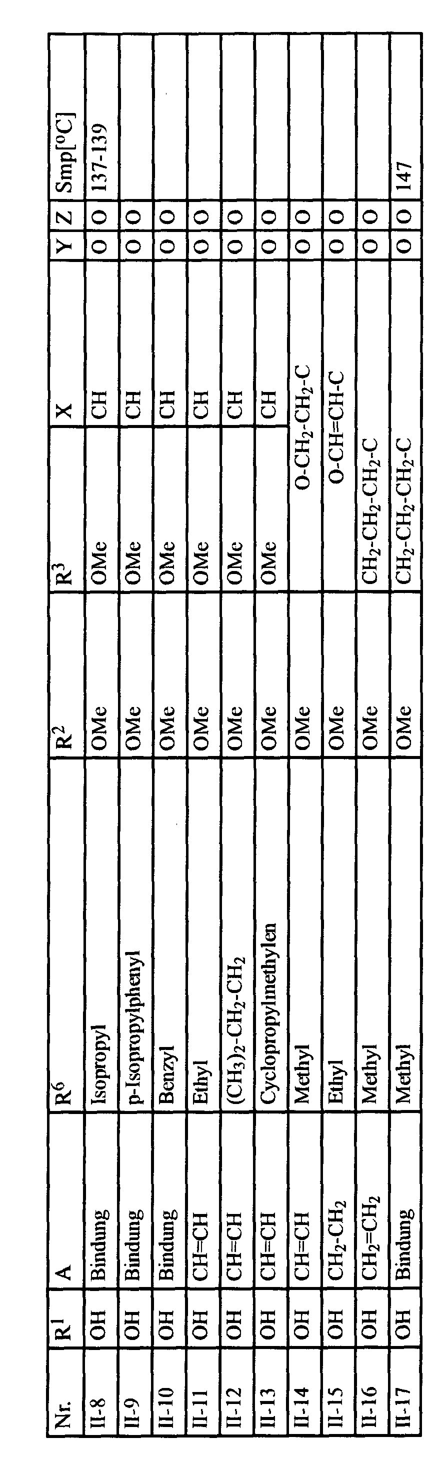

### Beispiel 35

Gemäß dem oben beschriebenen Bindungstest wurden für die nachfolgend aufgeführten Verbindungen Rezeptorbindungsdaten gemessen.

Die Ergebnisse sind in Tabelle 2 dargestellt.

### Tabelle 2

### Rezeptorbindungsdaten (Kᵢ-Werte)

| Verbindung | ET_{A} [nM] | ET_{B} [nM] |
|---|---|---|
| | | |
| I-2 | 6 | 34 |
| I-29 | 86 | 180 |
| I-5 | 12 | 160 |
| I-4 | 7 | 2500 |
| I-87 | 1 | 57 |
| I.89 | 86 | 9300 |
| I-103 | 0,4 | 29 |
| I-107 | 3 | 485 |
| I-12 | 19 | 1700 |
| I-26 | 23 | 2000 |
| I-23 | 209 | 1100 |
| I-47 | 150 | 1500 |
| I-60 | 33 | 970 |
| I-96 | 0,6 | 56 |
| | | |
| II-3 | 107 | 7300 |
| II-1 | 28 | 2300 |

## Patentansprüche

1. Carbonsäurederivate der Formel I 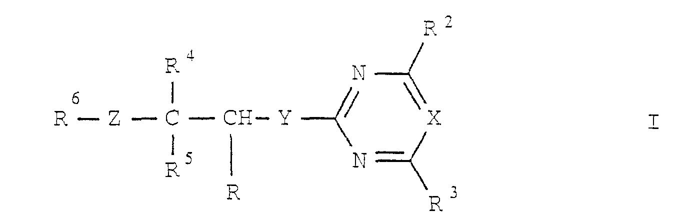 in der R, ein Tetrazol, oder eine Gruppe COOH bedeutet und die übrigen Substituenten folgende Bedeutung haben:
R² Wasserstoff, Hydroxy, NH₂, NH(C₁-C₄-Alkyl), N(C₁-C₄-Alkyl)₂, Halogen, C₁-C₄-Alkyl, C₁-C₄-Halogenalkyl, C₁-C₄-Alkoxy, C₁-C₄-Halogenalkoxy oder C₁-C₄-Alkylthio;
X Stickstoff oder CR¹⁴, worin R¹⁴ Wasserstoff oder C₁₋₅-Alkyl bedeutet oder CR¹⁴ zusammen mit CR³ einen 5- oder 6-gliedrigen Alkylen- oder Alkenylenring bildet, der durch eine oder zwei C₁₋₄-Alkylgruppen substituiert sein kann und worin jeweils eine Methylengruppe durch Sauerstoff, Schwefel, -NH oder -NC₁₋₄-Alkyl ersetzt sein kann;
R³ Wasserstoff, Hydroxy, NH₂, NH(C₁-C₄-Alkyl), N(C₁-C₄-Alkyl)₂, Halogen, C₁-C₄-Alkyl, C₁-C₄-Halogenalkyl C₁-C₄-Alkoxy, C₁-C₄-Halogenalkoxy, -NH-O-C₁₋₄-Alkyl, C₁-C₄-Alkylthio oder CR³ ist mit CR¹⁴ wie oben angegeben zu einem 5- oder 6-gliedrigen Ring verknüpft;
R⁴ und R⁵ Phenyl oder Naphthyl, die durch einen oder mehrere der folgenden Reste substituiert sein können: Halogen, Nitro, Cyano, Eydroxy, C₁-C₄-Alkyl, C₁-C₄-Halogenalkyl, C₁-C₄-Alkoxy, C₁-C₄-Halogenalkoxy, Phenoxy, C₁-C₄-Alkylthio, Amino, C₁-C₄-Alkylamino oder C₁-C₄-Dialkylamino; oder Phenyl oder Naphthyl, die orthoständig über eine direkte Bindung, eine Methylen-, Ethylen- oder Ethenylengruppe ein Sauerstoff- oder Schwefelatom oder eine SO₂-, NH- oder N-Alkyl-Gruppe miteinander verbunden sind;
oder C₃-C₇-Cycloalkyl;
wobei R⁴ die gleiche Bedeutung wie R⁵ hat ;
R⁶ Wasserstoff, C₁-C₈-Alkyl, C₃-C₆-Alkenyl, C₃-C₆-Alkinyl oder C₃-C₈-Cycloalkyl, wobei diese Reste jeweils ein- oder mehrfach substituiert sein können durch: Halogen, Nitro, Cyano, C₁-C₄-Alkoxy, C₃-C₆-Alkenyloxy, C₃-C₆-Alkinyloxy, C₁-C₄-Alkylthio, C₁-C₄-Halogenalkoxy, C₁-C₄-Alkylcarbonyl, C₁-C₄-Alkoxycarbonyl, C₃₋₈-Alkylcarbonylalkyl, C₁-C₄-Alkylamino, Di-C₁-C₄-alkylamino, Phenyl oder ein- oder mehrfach, durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio substituiertes Phenyl oder Phenoxy;
Phenyl oder Naphthyl, die jeweils durch einen oder mehrere der folgenden Reste substituiert sein können: Halogen, Nitro, Cyano, Hydroxy, Amino, C₁-C₄-Alkyl, C₁-C₄-Halogenalkyl, C₁-C₄-Alkoxy, C₁-C₄-Halogenalkoxy Phenoxy, C₁-C₄-Alkylthio, C₁-C₄-Alkylamino, C₁-C₄-Dialkylamino oder Dioxomethylen oder Dioxoethylen;
ein fünf- oder sechsgliedriger Heteroaromat, enthaltend ein bis drei Stickstoffatome und/oder ein Schwefel- oder Sauerstoffatom, welcher ein bis vier Halogenatome und/ oder einen bis zwei der folgenden Reste tragen kann: C₁-C₄-Alkyl, C₁-C₄-Halogenalkyl, C₁-C₄-Alkoxy,
C₁-C₄-Halogenalkoxy, C₁-C₄-Alkylthio, Phenyl, Phenoxy oder Phenylcarbonyl, wobei die Phenylreste ihrerseits ein bis fünf Halogenatome und/oder einen bis drei der folgenden Reste tragen können: C₁-C₄-Alkyl, C₁-C₄-Halogenalkyl, C₁-C₄-Alkoxy, C₁-C₄-Halogenalkoxy und/oder C₁-C₄-Alkylthio;
Y Sauerstoff;
z Schwefel, Sauerstoff, -SO-, -SO₂-.

2. Carbonsäurederivate nach Anspruch 1, wobei
R² = R³ = C₁-C₄-Alkyl oder C₁-C₄-Alkoxy,
X = N oder CH,
R⁴ = R⁵ = Phenyl,
R⁶ = C₁-C₈-Alkyl, das ein- oder mehrfach substituiert sein kann durch Halogen, Nitro, Cyano, C₁-C₄-Alkoxy, C₃-C₆-Alkenyloxy, C₃-C₆-Alkinyloxy, C₁-C₄-Alkylthio, C₁-C₄-Halogenalkoxy, C₁-C₄-Alkylcarbonyl, C₁-C₄-Alkoxycarbonyl, C₃₋₈-Alkylcarbonylalkyl, C₁-C₄-Alkylamino, Di-C₁-C₄-alkylamino, Phenyl oder ein- oder mehrfach, durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio substituiertes Phenyl oder Phenoxy,
Y = O,
z = O
bedeuten.

3. Carbonsäurederivate nach Anspruch 1 oder 2 in Form reiner Enantiomere.

4. Verwendung von Carbonsäurederivaten nach Anspruch 1 bis 3 zur Herstellung von Arzneimitteln.

5. Verwendung nach Anspruch 4 als Hemmstoffe für Endothelinrezeptoren.

6. Verwendung nach Anspruch 4 zur Behandlung von Erkrankungen, bei denen erhöhte Plasmaspiegel von Endothelin gefunden werden.

7. Verwendung nach Anspruch 4 für die Behandlung von Hypertonie, Myokardinfarkt, Angina Pectoris, Restenose nach Angioplastie.

8. Verwendung nach Anspruch 4 bis 7 für orale Verabreichung.

9. Carbonsäurederivale der Formel I wobei R ein zu coott hydrolysierbarer Rest bedentet und die übrigen Substituenten die gleiche Bedeutung wie in Anspruch 1 haben.

## Claims

1. A carboxylic acid derivative of the formula I 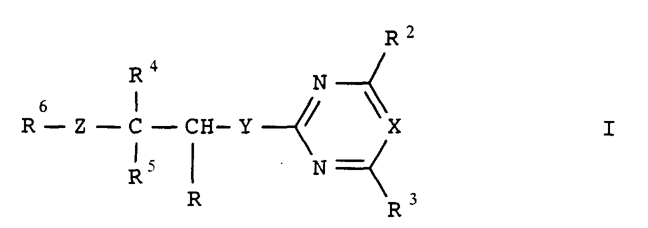 where R is a tetrazolyl or a COOH group, and the other substituents have the following meanings:
R² hydrogen, hydroxyl, NH₂, NH(C₁-C₄-alkyl), N(C₁-C₄-alkyl)₂, halogen, C₁-C₄-alkyl, C₁-C₄-haloalkyl, C₁-C₄-alkoxy, C₁-C₄-haloalkoxy or C₁-C₄-alkylthio;
X nitrogen or CR¹⁴ where R¹⁴ is hydrogen or C₁₋₅-alkyl, or CR¹⁴ forms together with CR³ a 5- or 6-membered alkylene or alkenylene ring which can be substituted by one or two C₁₋₄-alkyl groups and in which in each case a methylene group can be replaced by oxygen, sulfur, -NH or -NC₁₋₄-alkyl;
R³ hydrogen, hydroxyl, NH₂, NH(C₁-C₄-Alkyl), N(C₁-C₄-alkyl)₂, halogen, C₁-C₄-alkyl, C₁-C₄-haloalkyl, C₁-C₄-alkoxy, C₁-C₄-haloalkoxy, -NH-O-C₁₋₄-alkyl, C₁-C₄-alkylthio or CR³ is linked to CR¹⁴ as indicated above to give a 5- or 6-membered ring;
R⁴ and R⁵ phenyl or naphthyl, which can be substituted by one or more of the following radicals: halogen, nitro, cyano, hydroxyl, C₁-C₄-alkyl, C₁-C₄-haloalkyl, C₁-C₄-alkoxy, C₁-C₄-haloalkoxy, phenoxy, C₁-C₄-alkylthio, amino, C₁-C₄-alkylamino or C₁-C₄-dialkylamino; or
phenyl or naphthyl, which are connected together in the ortho positions via a direct linkage, a methylene, ethylene or ethenylene group, an oxygen or sulfur atom or an SO₂, NH or N-alkyl group
or C₃-C₇-cycloalkyl;
where R⁴ has the same meaning as R⁵;
R⁶ hydrogen, C₁-C₈-alkyl, C₃-C₆-alkenyl, C₃-C₆-alkynyl or C₃-C₈-cycloalkyl, where each of these radicals can be substituted one or more times by: halogen, nitro, cyano, C₁-C₄-alkoxy, C₃-C₆-alkenyloxy, C₃-C₆-alkynyloxy, C₁-C₄-alkylthio, C₁-C₄-haloalkoxy, C₁-C₄-alkylcarbonyl, C₁-C₄-alkoxycarbonyl, C₃₋₈-alkylcarbonylalkyl,
C₁-C₄-alkylamino, di-C₁-C₄-alkylamino, phenyl, or phenyl or phenoxy which is substituted one or more times by halogen, nitro, cyano, C₁-C₄-alkyl, C₁-C₄-haloalkyl, C₁-C₄-alkoxy, C₁-C₄-haloalkoxy or C₁-C₄-alkylthio;
phenyl or naphthyl, each of which can be substituted by one or more of the following radicals: halogen, nitro, cyano, hydroxyl, amino, C₁-C₄-alkyl, C₁-C₄-haloalkyl, C₁-C₄-alkoxy, C₁-C₄-haloalkoxy, phenoxy, C₁-C₄-alkylthio, C₁-C₄-alkylamino, C₁-C₄-dialkylamino, methylenedioxy or ethylenedioxy;
a five- or six-membered heteroaromatic moiety containing one to three nitrogen atoms and/or one sulfur or oxygen atom, which can carry one to four halogen atoms and/or one or two of the following radicals: C₁-C₄-alkyl, C₁-C₄-haloalkyl,
C₁-C₄-alkoxy, C₁-C₄-haloalkoxy, C₁-C₄-alkylthio, phenyl, phenoxy or phenylcarbonyl, it being possible for the phenyl radicals in turn to carry one to five halogen atoms and/or one to three of the following radicals: C₁-C₄-alkyl, C₁-C₄-haloalkyl, C₁-C₄-alkoxy, C₁-C₄-haloalkoxy and/or C₁-C₄-alkylthio;
Y oxygen;
Z sulfur, oxygen, -SO-, -SO₂-.

2. A carboxylic acid derivative as claimed in claim 1, where
R² = R³ - C₁-C₄-alkyl or C₁-C₄-alkoxy;
X = N or CH,
R⁴ = R⁵ = phenyl,
R⁶ = C₁-C₈-alkyl which can be substituted one or more times by: halogen, nitro, cyano, C₁-C₄-alkoxy, C₃-C₆-alkenyloxy, C₃-C₆-alkynyloxy, C₁-C₄-alkylthio, C₁-C₄-haloalkoxy, C₁-C₄-alkylcarbonyl, C₁-C₄-alkoxycarbonyl,
C₃₋₈-alkylcarbonylalkyl, C₁-C₄-alkylamino, di-C₁-C₄-alkylamino, phenyl, or phenyl or phenoxy which is substituted one or more times by halogen, nitro, cyano, C₁-C₄-alkyl, C₁-C₄-haloalkyl, C₁-C₄-alkoxy, C₁-C₄-haloalkoxy or C₁-C₄-alkylthio;
Y = 0,
Z O.

3. A carboxylic acid derivative as claimed in claim 1 or 2 in the form of pure enantiomers.

4. The use of carboxylic acid derivatives as claimed in claim 1 to 3 for producing drugs.

5. The use as claimed in claim 4 as inhibitors of endothelin receptors.

6. The use as claimed in claim 4 for treating disorders in which elevated plasma endothelin levels are found.

7. The use as claimed in claim 4 for treating hypertension, myocardialinfarction, angina pectoris, restenosis after angioplasty.

8. The use as claimed in claim 4 to 7 for oral administration.

9. A carboxylic acid derivative of the formula I where R is a radical which can be hydrolyzed to COOH, and the other substituents have the same meaning as in claim 1.

## Revendications

1. Dérivés d'acide carboxylique de formule I 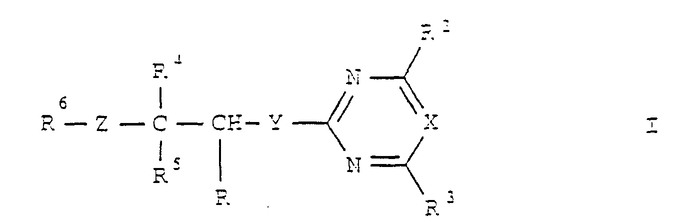 dans laquelle R représente un tétrazole ou un groupe COOH et les autres substituants ont la signification suivante:
R2 hydrogène, hydroxy, NH₂, NH(alkyle en C₁-C₄), N(alkyle en C₁-C₄)₂, halogéno, alkyle en C₁-C₄, halogénoalkyle en C₁-C₄, alcoxy en C₁-C₄, halgénoalcoxy en C₁-C₄ ou alkylthio en C₁-C₄;
X azote ou CR¹⁴, où R¹⁴ représente l'hydrogène ou un groupe alkyle en C₁₋₅, ou CR¹⁴ forme conjointement avec CR³ un cycle alkylène ou alcényle à 5 ou 6 chaînons, qui peut être substitué par un ou deux groupes alkyle en C₁₋₄ et dans lequel à chaque fois un groupe méthylène peut être remplacé par l'oxygène, le soufre, -NH ou -N(alkyle en C₁₋₄);
R3 hydrogène, NH₂, NH(alkyle en C₁-C₄), N(alkyle en C₁-C₄)₂, halogéno, alkyle en C₁-C₄, halogénoalkyle en C₁-C₄, alcoxy en C₁-C₄, halogénoalcoxy en C₁-C₄, -NH-O-alkyle en C₁-C₄, alkylthio en C₁-C₄ ou CR³ est lié avec CR¹⁴ comme indiqué plus haut pour former un cycle à 5 ou 6 chaînons;
R4 et R⁵ phényle ou naphtyle, qui peuvent être substitués par un ou plusieurs des restes suivants: halogéno, nitro, cyano, hydroxy, alkyle en C₁-C₄, halogénoalkyle en C₁-C₄, alcoxy en C₁-C₄, halogénoalcoxy en C₁-C₄, phénoxy, alkylthio en C₁-C₄, amino, alkylamino en C₁-C₄ ou (dialkyl en C₁-C₄)amino;
ou
phényle ou naphtyle, qui sont liés entre eux en position ortho par une liaison directe, un groupe méthylène, éthylène ou éthénylène, un atome d'oxygène ou de soufre, ou un groupe SO₂-, NH- ou N-alkyle;
ou cycloalkyle en C₃-C₇;
tandis que R⁴ a la même signification que R⁵;
R6 hydrogène, alkyle en C₁-C₈, alcényle en C₃-C₆, alcynyle en C₃-C₆ ou cycloalkyle en C₃-C₈, tandis que ces restes peuvent à chaque fois être substitués une ou plusieurs fois par: halogéno, nitro, cyano, alcoxy en C₁-C₄, alcényloxy en C₃-C₆, alcynyloxy en C₃-C₆, alkylthio en C₁-C₄, halogénoalcoxy en C₁-C₄, alkyl(C₁-C₄)carbonyle, alcoxy(C₁-C₄)carbonyle, alkylcarbonylalkyle en C₃-C₈, alkylamino en C₁-C₄, (dialkyl en C₁-C₄)amino, phényle ou phényle ou phénoxy substitué une ou plusieurs fois par halogéno, nitro, cyano, alkyle en C₁-C₄, halogénoalkyle en C₁-C₄, alcoxy en C₁-C₄, halogénoalcoxy en C₁-C₄ ou alkylthio en C₁-C₄;
phényle ou naphtyle, qui peuvent être substitués à chaque fois par un ou plusieurs des restes suivants: halogéno, nitro, cyano, hydroxy, amino, alkyle en C₁-C₄, halogénoalkyle en C₁-C₄, alcoxy en C₁-C₄, halogénoalcoxy en C₁-C₄, phénoxy, alkylthio en C₁-C₄, alkylamino en C₁-C₄, (dialkyl en C₁-C₄)amino ou dioxométhylène ou dioxoéthylène;
un groupe hétéroaromatique à cinq ou six chaînons, contenant un à trois atomes d'azote et/ou un atome de soufre ou d'oxygène, qui peut porter un à quatre atomes d'halogène et/ou un à deux des restes suivants: alkyle en C₁-C₄, halogénoalkyle en C₁-C₄, alcoxy en C₁-C₄, halogénoalcoxy en C₁-C₄, akylthio en C₁-C₄, phényle, phénoxy ou phénylcarbonyle, tandis que les restes phényle peuvent à leur tour porter un à cinq atomes d'halogène et/ou un à trois des restes suivants: alkyle en C₁-C₄, halogénoalkyle en C₁-C₄, alcoxy en C₁-C₄, halogénoalcoxy en C₁-C₄ et/ou alkylthio en C₁-C₄;
Y oxygène;
Z soufre, oxygène, -SO-, -SO₂-.

2. Dérivés d'acide carboxylique selon la revendication 1, dans lesquels
R² = R³ = alkyle en C₁-C₄ ou alcoxy en C₁-C₄,
X = N ou CH,
R⁴ = R⁵ = phényle,
R6 = alkyle en C₁-C₈, qui peut être substitué une ou plusieurs fois par halogéno, nitro, cyano, alcoxy en C₁-C₄, alcényloxy en C₃-C₆, alcynyloxy en C₃-C₆, alkylthio en C₁-C₄, halogénoalcoxy en C₁-C₄, alkyl(C₁-C₄)carbonyle, alcoxy(C₁-C₄)-carbonyle, alkyl-carbonylalkyle en C₃-C₈, alkylamino en C₁-C₄, (dialkyl en C₁-C₄)amino, phényle ou phényle ou phénoxy substitué une ou plusieurs fois par halogéno, nitro, cyano, alkyle en C₁-C₄, halogénoalkyle en C₁-C₄, alcoxy en C₁-C₄, halogénoalcoxy en C₁-C₄ ou alkylthio en C₁-C₄,
Y = O,
Z = O.

3. Dérivés d'acide carboxylique selon la revendication 1 ou 2 sous forme d'énantiomères purs.

4. Utilisation de dérivés d'acide carboxylique selon la revendication 1 à 3 pour la préparation de médicaments.

5. Utilisation selon la revendication 4 à titre de substances inhibant les récepteurs d'endothéline.

6. Utilisation selon la revendication 4 pour le traitement de maladies, dans lesquelles on trouve une teneur élevée d'endothéline dans le plasma.

7. Utilisation selon la revendication 4 pour le traitement de l'hypertonie, de l'infarctus du myocarde, de l'angine de poitrine, de la resténose après angioplastie.

8. Utilisation selon la revendication 4 à 7 pour administration orale.

9. Dérivés d'acide carboxylique de formule I, dans lesquels R représente un reste hydrolysable en COOH et les autres substituants ont la même signification que dans la revendication 1.
